# EUROPEAN PATENT SPECIFICATION

(11) **EP 2 178 455 B1**
(45) Date of publication and mention of the grant of the patent: **06.05.2015**
(21) Application number: 08792283.7
(22) Date of filing: 31.07.2008
(51) Int. Cl.: A61B 18/16

(54) **TREATMENT DEVICE FOR LIVING TISSUE**
VORRICHTUNG ZUR BEHANDLUNG VON LEBENDEM GEWEBE
DISPOSITIF DE TRAITEMENT POUR UN TISSU VIVANT

(30) Priority: 14.08.2007 US 838403
(43) Date of publication of application: 28.04.2010
(73) Proprietor: Olympus Medical Systems Corp., Tokyo 151-0072 (JP)
(72) Inventor: TAKASHINO, Tomoyuki, Tokyo 192-8512 (JP); NAGASE, Toru, Tokyo 192-8512 (JP); IIDA, Koji, Tokyo 192-8512 (JP); WAKAMATSU, Mai, Tokyo 192-8512 (JP)
(74) Representative: Gunzelmann, Rainer
(86) International application number: PCT/JP2008/064176
(87) International publication number: WO 2009/022614

(56) References cited:
- EP-A1- 0 878 168
- US-A1- 2006 047 331
- US-A1- 2006 064 086
- US-A1- 2007 102 472

## Description

### Technical Field

The present invention relates to a treatment device for a living tissue.

### Background Art

It is increasingly apparent in general that, in the case of jointing living tissues to each other, the regeneration and/or restoration of the living tissues progress earlier in the vicinity of a part where the jointed living tissues are in contact with each other. If the living tissues in the vicinity of the jointed living tissues are in contact with each other, the living tissues in contact will be brought into the process of regeneration and/or restoration in- a few days.

Therefore, there is a demand for creation of a treatment device, which can keep living tissues in the vicinity of jointed living tissues in contact with each other, so that the living tissues can be regenerated and/or restored earlier.

EP 0 878 168 A1 discloses an electrosurgical instrument provided for cauterization and/or welding of tissue of varying impedance, thickness and vascularity especially in the performance of endoscopic procedures. The instrument compresses the tissue between one pole of a bipolar energy source located on one interfacing surface, and a second interfacing surface applying pressure in a predetermined range. A second pole is located on one of the two interfacing surfaces. According to one embodiment, the first and second poles comprise a series of electrically connected electrodes staggered along the length of the knife channel with insulating material in between staggered electrodes.

### Disclosure of Invention

An object of the present invention is to provide a treatment device, which can keep living tissues in the vicinity of jointed living tissues in contact with each other, so that the living tissues can be regenerated and/or restored earlier.

An object of the present invention is to provide a treatment device capable of maintaining the closely contacted state of the living tissues located in the vicinity of the living tissues jointed, and the living tissues are able to be regenerated and/or restored earlier.

According to a first aspect of the present invention, there is provided a treatment device according to claim 1.

### Brief Description of Drawings

FIG. 1A is a schematic view showing a treatment system according to a first embodiment of the present invention;
FIG. 1B is a schematic view when the treatment system according to the first embodiment is used to perform a bipolar-type treatment;
FIG. 2A is a schematic longitudinal sectional view showing a shaft and a closed state of a first holding member and a second holding member of a holding section in an electro-surgical device according to the first embodiment;
FIG. 2B is a schematic longitudinal sectional view showing the shaft and an opened state of the first holding member and the second holding member of the holding section in the electro-surgical device according to the first embodiment;
FIG. 3A is a schematic plan view showing the first holding member on a side close to the second holding member in the holding section of the electro-surgical device according to the first embodiment;
FIG. 3B is a schematic longitudinal sectional view showing the first holding member taken along the line 3B-3B depicted in FIG. 3A in the holding section of the electro-surgical device according to the first embodiment;
FIG. 3C is a schematic traverse sectional view cut along the 3C-3C line depicted in FIG. 3A, showing the first holding member in the holding section of the electro-surgical device according to the first embodiment;
FIG. 4A is a schematic perspective view cut along the 4A-4A line depicted in FIG. 4C mentioned later, showing two tracts of the small intestine that are anastomosed to each other;
FIG. 4B is a schematic view showing a part denoted by reference number 4B in FIG. 4A in an enlarged manner;
FIG. 4C is a schematic view showing the sealed ends of the two tracts of the small intestine after anastomosed;
FIG. 4D is a schematic view as a modification of FIG. 4B, showing the part denoted by reference number 4B in FIG. 4A in an enlarged manner;
FIG. 5A is a schematic plan view showing the first holding member on a side close to the second holding member in the holding section of the electro-surgical device according to a first modification of the first embodiment;
FIG. 5B is a schematic plan view showing the first holding member on a side close to the second holding member in the holding section of the electro-surgical device according to a second modification of the first embodiment;
FIG. 6A is a schematic view when a treatment system according to the first embodiment is used to perform a bipolar-type treatment;
FIG. 6B is a schematic view when the treatment system according to the first embodiment is used to perform a monopolar-type treatment;
FIG. 6C is a schematic view when the treatment system according to the first embodiment is used to perform a monopolar-type treatment;
FIG. 7 is a schematic view showing a modification of the treatment system according to the first embodiment;
FIG. 8 is a schematic plan view showing the first holding member on a side close to the second holding member in the holding section of the electro-surgical device according to a second embodiment of the present invention;
FIG. 9A is a schematic plan view showing the first holding member on a side close to the second holding member in the holding section of the electro-surgical device according to a first modification of the second embodiment;
FIG. 9B is a schematic plan view showing the first holding member on a side close to the second holding member in the holding section of the electro-surgical device according to a second modification of the second embodiment;
FIG. 10 is a schematic plan view showing the first holding member on a side close to the second holding member in the holding section of the electro-surgical device according to a third embodiment of the present invention;
FIG. 11A is a schematic plan view showing the first holding member on a side close to the second holding member in the holding section of the electro-surgical device according to a first modification of the third embodiment;
FIG. 11B is a schematic plan view showing the first holding member on a side close to the second holding member in the holding section of the electro-surgical device according to a second modification of the third embodiment;
FIG. 12A is a schematic longitudinal sectional view showing a shaft and a closed state of a first holding member and a second holding member of a holding section in an electro-surgical device according to the fourth embodiment of the present invention;
FIG. 12B is a schematic longitudinal sectional view showing the shaft and an opened state of the first holding member and the second holding member of the holding section in the electro-surgical device according to the fourth embodiment;
FIG. 13A is a schematic plan view showing the first holding member on a side close to the second holding member in the holding section of the electro-surgical device according to the fourth embodiment of the present invention;
FIG. 13B is a schematic longitudinal sectional view cut along the 13B-13B line depicted in FIG. 13A, showing the first holding member of the holding section of the electro-surgical device according to the fourth embodiment;
FIG. 14A is a schematic plan view showing the first holding member on a side close to the second holding member in the holding section of the electro-surgical device according to a first modification of the fourth embodiment of the present invention;
FIG. 14B is a schematic longitudinal sectional view cut along the 14B-14B line depicted in FIG. 14A, showing the first holding member of the holding section of the electro-surgical device according to a first modification of the fourth embodiment;
FIG. 14C is a schematic longitudinal sectional view cut along the 14B-14B line depicted in FIG. 14A, showing the first holding member of the holding section of the electro-surgical device according to a second modification of the fourth embodiment;
FIG. 15 is a schematic plan view showing the first holding member on a side close to the second holding member in the holding section of the electro-surgical device according to a fifth embodiment of the present invention;
FIG. 16A is a schematic plan view showing the first holding member on a side close to the second holding member in the holding section of the electro-surgical device according to a sixth embodiment of the present invention;
FIG. 16B is a schematic longitudinal sectional view cut along the 16B-16B line depicted in FIG. 16A, showing the first holding member of the holding section of the electro-surgical device according to the sixth embodiment;
FIG. 16C is a schematic longitudinal sectional view cut along the 16B-16B line depicted in FIG. 16A, showing the first holding member of the holding section of the electro-surgical device according to a first modification of the sixth embodiment;
FIG. 16D is a schematic longitudinal sectional view cut along the 16B-16B line depicted in FIG. 16A, showing the first holding member of the holding section of the electro-surgical device according to a second modification of the sixth embodiment;
FIG. 17A is a schematic plan view showing the first holding member to discharge staples on a side close to the second holding member in the holding section of a treatment device according to a seventh embodiment, not of the present invention;
FIG. 17B is a schematic plan view showing the second holding member to fold the legs of the staples on a side close to the first holding member in the holding section of the treatment device according to the seventh embodiment;
FIG. 18 is a schematic longitudinal sectional view cut along the 18A-18A line depicted in FIG. 17A, showing the first holding member of the holding section of the electro-surgical device according to the seventh embodiment;
FIG. 19 is a schematic plan view showing the first holding member to discharge staples on a side close to the second holding member in the holding section of a treatment device according to an eighth embodiment, not of the present invention;
FIG. 20 is a schematic view showing a treatment system according to a ninth embodiment of the present invention;
FIG. 21A is a schematic longitudinal sectional view showing a state in which a main body side holding portion engages with a detachable side holding portion and the detachable side holding portion is disposed separated from the main body side holding portion in an electro-surgical device according to the ninth embodiment;
FIG. 21B is a schematic longitudinal sectional view showing a state in which the main body side holding portion engages with the detachable side holding portion and the detachable side holding portion is disposed close to the main body side holding portion in the electro-surgical device according to the ninth embodiment;
FIG. 21C is an enlarged schematic view showing a part of main body side holding portion denoted by reference number 21C in the electro-surgical device according to the ninth embodiment;
FIG. 21D is a schematic view showing the surface of the main body side holding portion of the electro-surgical device according to the ninth embodiment;
FIG. 22A is a schematic view showing the surface of a main body side holding portion of an electro-surgical device according to a tenth embodiment of the present invention;
FIG. 22B is a schematic view showing the surface of a main body side holding portion of an electro-surgical device according to an eleventh embodiment of the present invention;
FIG. 23A is a schematic view showing a separated state of a main body side holding portion and a detachable side holding portion of an electro-surgical device according to a twelfth embodiment of the present invention;
FIG. 23B is a schematic longitudinal sectional view showing a state in which the main body side holding portion engages with the detachable side holding portion in the electro-surgical device according to the twelfth embodiment;
FIG. 23C is a schematic view showing the surface of the main body side holding portion of the electro-surgical device according to the twelfth embodiment;
FIG. 24A is a schematic longitudinal sectional view showing a state in which a main body side holding portion engages with a detachable side holding portion and the detachable side holding portion is disposed separated from the main body side holding portion in an electro-surgical device according to a thirteenth embodiment, not of the present invention;
FIG. 24B is a schematic view showing the surface of the main body side holding portion of the electro-surgical device according to the thirteenth embodiment; and
FIG. 24C is a schematic view showing the surface of the main body side holding portion of the electro-surgical device according to a modification of the thirteenth embodiment.

### Best Mode for Carrying Out the Invention

The best mode for carrying out the present invention will now be explained hereinafter with reference to the accompanying drawings.

### [First Embodiment]

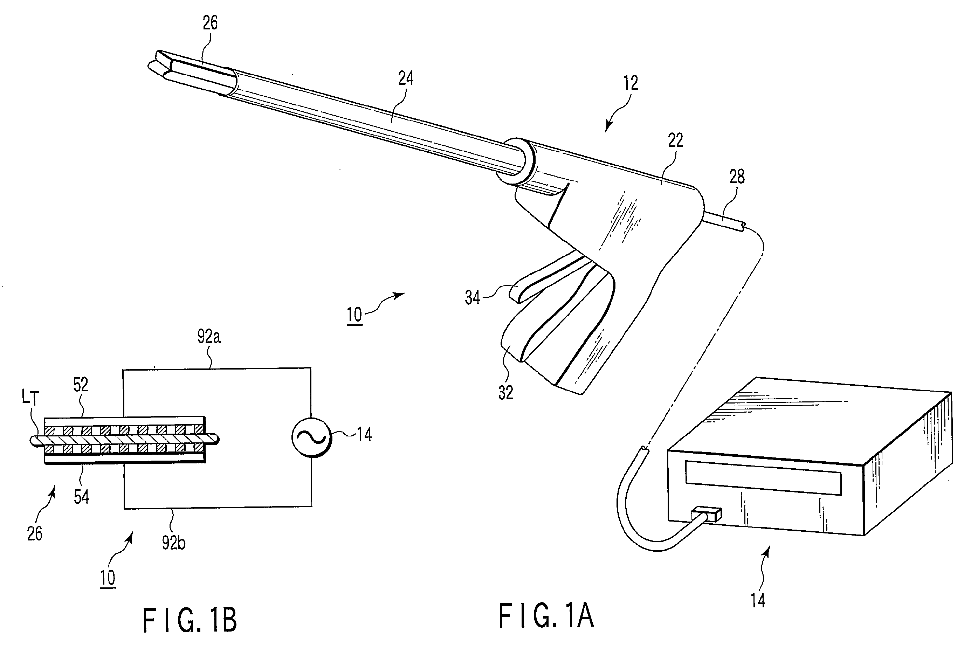

A first embodiment will be explained with reference to FIGS. 1A to 7. Here, as an example of an energy treatment device, a linear-type bipolar electro-surgical device 12 which performs a treatment through, for example, an abdominal wall will be described.

As shown in FIGS. 1A and 1B, a treatment system 10 includes the electro-surgical device (treatment device for curing) 12 and an energy source 14.

The electro-surgical device 12 includes a handle 22, a shaft 24 and an openable/closable holding section 26. The handle 22 is connected with the energy source 14 via a cable 28. The energy source 14 is connected to a foot switch and a hand switch (not shown). Therefore, these foot and hand switches are operated by an operator to switch on or off the supply of energy from the energy source 14 to the electro-surgical device 12.

The handle 22 is substantially formed into an L-shape. The shaft 24 is disposed on one end of the handle 22. The cable 28 is extended from a proximal side of the handle 22 disposed coaxially with the shaft 24.

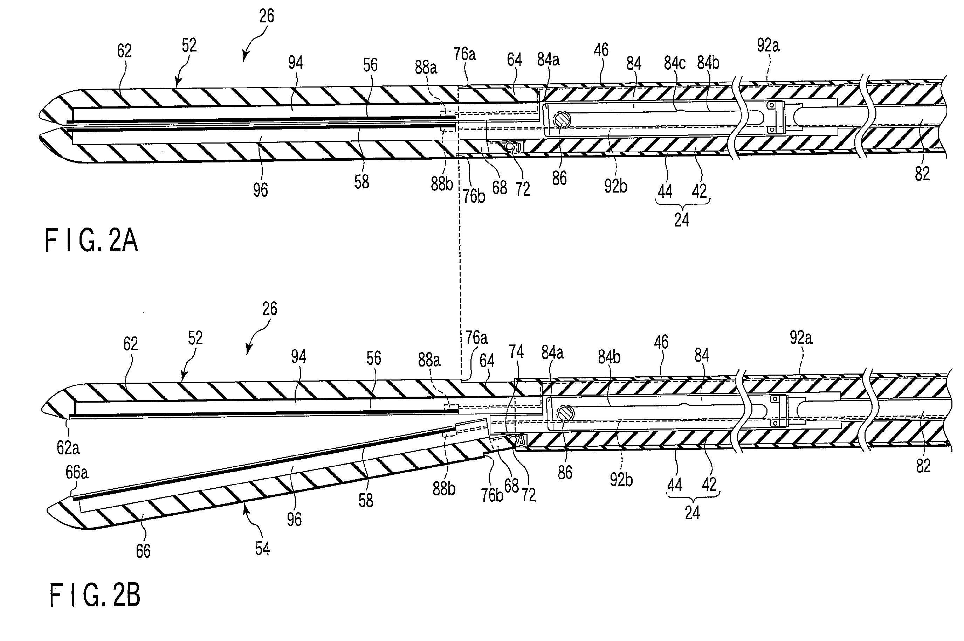

On the other hand, the other end of the handle 22 is a grip held by the operator. The handle 22 includes a holding section opening/closing knob 32 arranged on the other end of the handle 22. The holding section opening/closing knob 32 is connected to a proximal end of a sheath 44 (see FIGS. 2A and 2B) described later of the shaft 24 substantially at the center of the handle 22. When the holding section opening/closing knob 32 is allowed to come close to or come away from the other end of the handle 22, the sheath 44 moves along an axial direction of the shaft 24. The handle 22 is provided with a cutter driving knob 34 disposed along a holding section opening/closing knob 32 to move a cutter 84 described later.

As shown in FIGS. 2A and 2B, the shaft 24 includes a cylindrical member 42 and the sheath 44 sildably disposed outside the cylindrical member 42. A proximal end of the cylindrical member 42 is fixed to the handle 22 (see FIG. 1A). The sheath 44 is slidable along an axial direction of the cylindrical member 42.

Outside the cylindrical member 42, a concave portion 46 is formed along the axial direction of the cylindrical member 42. The concave portion 46 is provided with a first conducting line 92a connected to a first high-frequency electrode (applying portion) 56 described later. A second conducting line 92b connected to a second high-frequency electrode (applying portion) 58 described later is passed through the cylindrical member 42.

It is to be noted that the first high-frequency electrode 56 is electrically connected with a first electrode connector 88a. The first electrode connector 88a is connected with the cable 28 extended from the handle 22 via a first energization line 92a. The second high-frequency electrode 58 is electrically connected with a second electrode connector 88b. The second electrode connector 88b is connected with the cable 28 extended from the handle 22 via a second energization line 92b.

A driving rod 82 is movably disposed along an axial direction of the cylindrical member 42 in the cylindrical member of the shaft 24. A distal end of the driving rod 82 is provided with a thin-plate-like cutter 84 (an auxiliary curative device). Therefore, when the cutter driving knob 34 is operated, the cutter 84 moves via the driving rod 82.

A distal end of the cutter 84 is provided with a blade 84a, and the distal end of the driving rod 82 is fixed to a proximal end of the cutter 84. A longitudinal groove 84b is formed between the distal end and the proximal end of the cutter 84. In the longitudinal groove 84b, the movement regulation pin 86 extending in a direction crossing the axial direction of the shaft 24 at right angles is fixed to the cylindrical member 42 of the shaft 24. Therefore, the longitudinal groove 84b of the cutter 84 moves along the movement regulation pin 86. In this case, the cutter 84 linearly moves. At this time, the cutter 84 is disposed along cutter guide grooves (flow channels, fluid discharge grooves) 94, 96 described later of a first holding member 52 and a second holding member 54.

It is to be noted that engagement portions 84c which engage with the movement regulation pin 86 and which control the movement of the cutter 84 are formed on one end of the longitudinal groove 84b of the cutter 84, the other end and between one end and the other end.

As shown in FIGS. 1A, 2A, and 2B, the holding section 26 is disposed at a distal end of the shaft 24. As shown in FIGS. 2A and 2B, the holding section 26 includes the first holding member 52, the second holding member 54, the first high-frequency electrode 56 as an applying portion or an energy applying portion, and the second high-frequency electrode 58 as another applying portion or another energy applying portion.

It is preferable that the first holding member 52 and the second holding member 54 entirely have insulating properties, respectively. The first holding member 52 integrally includes a first holding portion main body (hereinafter referred to mainly as the main body) 62 provided with the first high-frequency electrode 56 and a base portion 64 disposed at a proximal end of the main body 62. The second holding member 54 integrally includes a second holding portion main body 66 provided with the second high-frequency electrode 58 and a base portion 68 disposed at a proximal end of the main body 66.

The base portion 64 of the first holding member 52 is fixed to a distal end of the cylindrical member 42 of the shaft 24. On the other hand, the base portion 68 of the second holding member 54 is rotatably supported at the distal end of the cylindrical member 42 of the shaft 24 by a support pin 72 disposed in a direction crossing the axial direction of the shaft 24 at right angles. The second holding member 54 can rotate around an axis of the support pin 72 to open or close with respect to the first holding member 52. Moreover, the second holding member 54 is urged so as to open with respect to the first holding member 52 by an elastic member 74 such as a leaf spring.

Outer surfaces of the main bodies 62, 66 of the first holding member 52 and the second holding member 54 are formed into smooth curved surfaces. Similarly, outer surfaces of the base portions 64, 68 of the first holding member 52 and the second holding member 54 are also formed into smooth curved surfaces. While the second holding member 54 is closed with respect to the first holding member 52, sections of the main bodies 62, 66 of the holding members 52, 54 are formed to be substantially circular or elliptical. When the second holding member 54 is closed with respect to the first holding member 52, holding surfaces 62a, 66a of the main bodies 62, 66 of the first and second holding members 52, 54 are opposite to each other, and the base portions 64, 68 are formed to be cylindrical. In this state, a diameter of each of the proximal ends of the main bodies 62, 66 of the first holding member 52 and the second holding member 54 is formed to be larger than a diameter of each of the base portions 64, 68. Moreover, stepped portions 76a, 76b are formed between the main bodies 62, 66 and the base portions 64, 68, respectively.

Here, in the first holding member 52 and the second holding member 54, while the second holding member 54 is closed with respect to the first holding member 52, a substantially circular or elliptical outer peripheral surface formed by combining the base portions 64, 68 of the holding members 52, 54 is substantially the same plane as that of an outer peripheral surface of the distal end of the cylindrical member 42, or a diameter of the outer peripheral surface is formed to be slightly larger than that of the outer peripheral surface of the distal end of the cylindrical member 42. Therefore, the sheath 44 can be slid with respect to the cylindrical member 42 to cover the base portions 64, 68 of the first holding member 52 and the second holding member 54 with a distal end of the sheath 44. In this state, as shown in FIG. 2A, the first holding member 52 and the second holding member 54 close against an urging force of the elastic member 74. On the other hand, the sheath 44 is slid toward the proximal end of the cylindrical member 42 from the state in which the base portions 64, 68 of the first holding member 52 and the second holding member 54 are covered with the distal end of the sheath 44. In this case, as shown in FIG. 2B, the second holding member 54 is opened with respect to the first holding member 52 by the urging force of the elastic member 74.

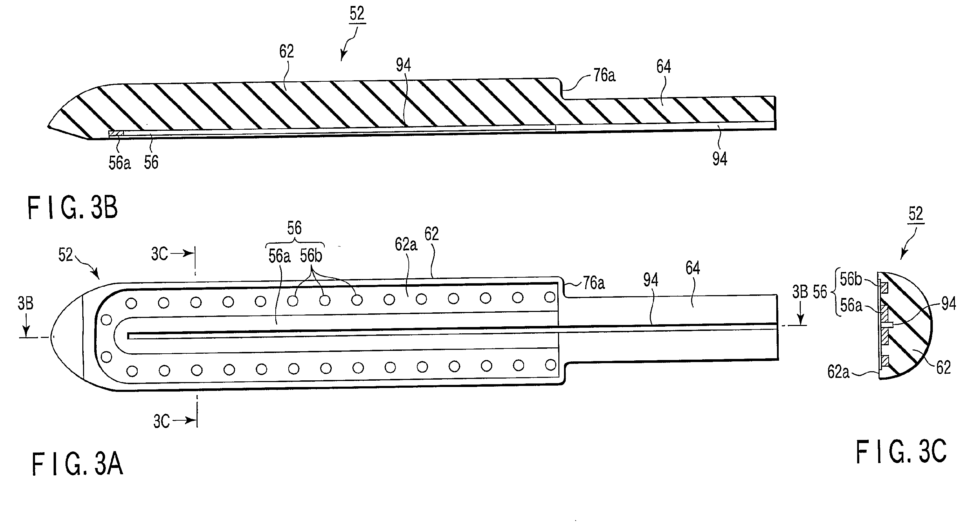

As shown in FIGS. 3A to 3C, the first high-frequency electrode 56 is disposed in the main body 62 of the first holding member 52. As shown in FIGS. 3A and 3C, the first high-frequency electrode 56 includes a seamlessly and continuously formed continuous electrode (sealing member, first jointing member) 56a, and a plurality of discrete electrodes (maintaining member, second jointing member) 56b discretely disposed outside the continuous electrode 56a.

The continuous electrode 56a is continuously formed into, for example, a substantially U-shape to have two ends at the proximal ends of the main body 62 of the first holding member 52. It is to be noted that a distance between proximal ends of the continuous electrode 56a is substantially equal to the width of the cutter guide groove 94 described later (see FIGS. 3A and 3C) in this embodiment, but the distance between proximal ends of the continuous electrode 56a can be suitably set. That is, the continuous electrode 56a may be provided at a distance from an edge of the cutter guide groove 94.

On the other hand, the plurality of discrete electrodes 56b of the same shape are arranged at substantially equal intervals along a substantially U-shaped virtual track. Each of the discrete electrode 56b is formed to be, for example, circular. The discrete electrodes 56b are arranged to have a predetermined space therebetween, and each of the discrete electrodes 56b is disposed at a distance from the continuous electrode 56a. The discrete electrodes 56b are positioned to allow a living tissue between the discrete electrode 56b and discrete electrodes 58b described later to be denatured by heat when a treatment is performed, but to maximally prevent the denaturation of a living tissue between the discrete electrodes 56b due to heat and also maximally prevent the denaturation of a living tissue between the discrete electrodes 56b and the continuous electrode 56a.

The cutter guide groove 94 for passing the cutter 84 therethrough is formed in the main body 62 and the base portion 64 of the first holding member 52. This cutter guide groove 94 is formed along the axial direction of the shaft 24. Thus, the cutter 84 is movable within the first holding member 52 along the cutter guide groove 94. The cutter 84 is also movable within the second holding member 54 along the cutter guide groove 96.

Furthermore, the second high-frequency electrode 58 is also disposed in the second holding member 54 symmetrically with the first holding member 52. This is omitting a description thereof in detail.

In addition, although not shown, reference number 58a is given to a continuous electrode of the second high-frequency electrode 58 for convenience, and reference number 58b is given to discrete electrodes thereof, so that the following function will be described.

A function of the treatment system 10 according to this embodiment will now be explained.

As shown in FIG. 2A, in a state where the second holding member 54 is closed with respect to the first holding member 52, the holding section 26 and the shaft 24 of the electro-surgical device 12 are inserted into, e.g., an abdominal cavity through an abdominal wall. The holding section 26 of the electro-surgical device 12 is opposed to a living tissue as a treatment target.

The holding section opening/closing knob 32 of the handle 22 is operated to hold (grasp) the living tissue as a treatment target by using the first holding member 52 and the second holding member 54. At this time, the sheath 44 is moved toward a proximal end side of the shaft 24 with respect to the cylindrical member 42. A space between the base portions 64, 68 cannot remain cylindrical due to an urging force of the elastic member 74, and the second holding member 54 is then opened with respect to the first holding member 52.

Moreover, the living tissue as a treatment target is arranged between the first high-frequency electrode 56 of the first holding member 52 and the second high-frequency electrode 58 of the second holding member 54. In this state, the holding section opening/closing knob 32 of the handle 22 is operated. At this time, the sheath 44 is moved to a distal end side of the shaft 24 with respect to the cylindrical member 42. The base portions 64, 68 are closed to form the cylindrical shape therebetween against the urging force of the elastic member 74 by using the sheath 44. Therefore, the first holding member main body 62 integrally formed on the base portion 64 and the second holding member main body 66 integrally formed on the base portion 68 are closed. That is, the second holding member 54 is closed with respect to the first holding member 52. Therefore, the living tissue L_{T} as a treatment target is grasped between the first holding member 52 and the second holding member 54.

At this time, the living tissue as a treatment target is in contact with both the first high-frequency electrode 56 provided on the first holding member 52 and the second high-frequency electrode 58 provided on the second holding member 54. A surrounding tissue of the living tissue L_{T} as a treatment target is closely contacted with both a holding surface 62a of the main body 62 of the first holding member 52 and a holding surface 66a of the main body 66 of the second holding member 54.

In this state, the foot switch or the hand switch is operated. Energy is respectively applied to the first high-frequency electrode 56 and the second high-frequency electrode 58 from the energy source 14 through the cable 28, the first and second energization lines 92a, 92b, and the first and second energization connectors 88a, 88b.

The first high-frequency electrode 56 passes a high-frequency current between this first high-frequency electrode 56 and the second high-frequency electrode 58 via the living tissue L_{T} as a treatment target. Thus, the living tissue L_{T} between the first high-frequency electrode 56 and the second high-frequency electrode 58 is heated. Then, the living tissue L_{T} is denatured in a continuous manner (in a substantially U-shaped state) by the continuous electrodes 56a, 58a of the first and second high-frequency electrodes 56, 58. Moreover, the living tissue L_{T} between the discrete electrodes 56b, 58b is discretely denatured by the discrete electrodes 56b, 58b of the first and second high-frequency electrodes 56, 58.

When a treatment is finished, the operation of the foot switch or the hand switch is stopped. Then, the supply of energy from the energy source 14 to the first high-frequency electrode 56 and the second high-frequency electrode 58 is stopped.

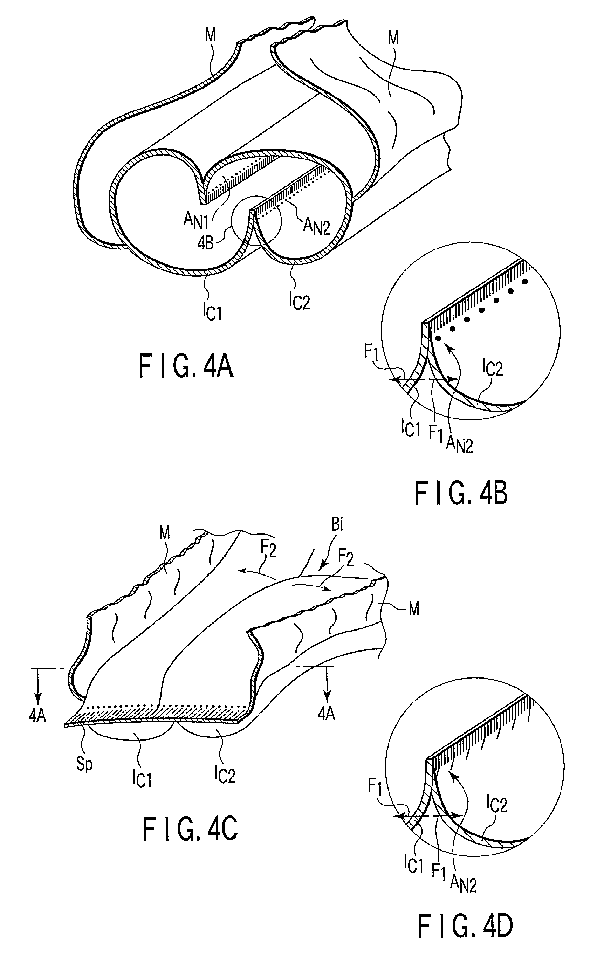

Here, a case will be described where the treatment system 10 having such a function is used to anastomose, for example, intestinal tracts I_{C1} and I_{C2} of the small intestine to each other as shown in FIGS. 4A to 4C.

The pair of intestinal tracts I_{C1} and I_{C2} provided side by side is held by the holding surfaces 62a, 66a of the first and second holding members 52, 54 so that wall surfaces of these intestinal tracts I_{C1} and I_{C2} are caught in between. In this state, energy is supplied to the first and second high-frequency electrodes 56, 58. Then, the intestinal tracts I_{C1} and I_{C2} held between the continuous electrode 56a of the first holding member 52 and the continuous electrode 58a of the second holding member 54 are heated and denatured. Thus, the wall surfaces of the intestinal tracts I_{C1} and I_{C2} are continuously denatured. Consequently, the intestinal tracts I_{C1} and I_{C2} are jointed to each other.

Furthermore, simultaneously with the jointing through the denaturation of the living tissues by the continuous electrodes 56a, 58a, the intestinal tracts I_{C1} and I_{C2} between the discrete electrode 56b of the first holding member 52 and the discrete electrode 58b of the second holding member 54 are denatured and jointed to each other. Thus, the living tissues of the intestinal tracts I_{C1} and I_{C2} are discretely denatured and jointed to each other.

Then, after the supply of energy to the first and second high-frequency electrodes 56, 58 is stopped, the cutter driving knob 34 shown in FIG. 1 is operated to move the cutter 84 forward along the cutter guide grooves 94, 96 from the state shown in FIG. 2A while the intestinal tracts I_{C1} and I_{C2} are gripped. As the cutter 84 moves forward, parts denatured and jointed to each other by the continuous electrodes 56a, 58a are cut. Then, the cutter 84 cuts inner sides of the parts denatured in a substantially U shape by the continuous electrodes 56a, 58a, up to the vicinity of distal ends thereof. Thus, parts of the wall surfaces of the intestinal tracts I_{C1} and I_{C2} which are sealed in a substantially U shape are cut, so that the wall surfaces of the intestinal tracts I_{C1} and I_{C2} are brought into communication with each other. That is, the wall surfaces of the intestinal tracts I_{C1} and I_{C2} are anastomosed to each other.

In this state, the first holding member 52 is opened with respect to the second holding member 54. At this time, there are formed a first anastomosed part A_{N1} on the side of a mesentery M, and a second anastomosed part A_{N2} opposite to the side where the mesentery M is located. For example, as shown in FIG. 4B, a continuously jointed outer portion of the second anastomosed part A_{N2} is discretely denatured.

Furthermore, energy is provided while the second holding member 54 is closed with respect to the first holding member 52 to hold the ends of the intestinal tracts I_{C1} and I_{C2}. Thus, as shown in FIG. 4C, the ends of the intestinal tracts I_{C1} and I_{C2} are denatured due to heat by the high-frequency electrodes 56, 58 and sealed. That is, a seal portion Sp is formed at the ends of the intestinal tracts I_{C1} and I_{C2}. At this time, the state of a section along the 4A-4A line depicted in FIG. 4C is as schematically shown in FIG. 4A. Thus, the intestinal tracts I_{C1} and I_{C2} are anastomosed to each other so that their ends are sealed at the seal portion S_{P}.

It is to be noted that extra parts of the seal portion Sp are cut by, for example, the cutter 84. At this time, parts around the continuously jointed portion of the sealed ends (seal portion Sp) of the intestinal tracts I_{C1} and I_{C2} are discretely denatured as shown in FIG. 4B. That is, the living tissues between the parts of the intestinal tracts I_{C1} and I_{C2} denatured and jointed by the discrete electrodes 56b, 58b are not denatured. Thus, the living tissues of the intestinal tracts I_{C1} and I_{C2} which are not denatured are in contact with (pressed against) each other around (in the vicinity of) the part where the living tissues are jointed by the discrete electrodes 56b, 58b.

Therefore, force acts on the first anastomosed part A_{N1} on the side of the mesentery M in such a direction as to cause the intestinal tracts I_{C1} and I_{C2} to be closely contacted with each other. Then, force functions in the part where the living tissues are denatured by the discrete electrodes 56b, 58b so that the living tissues are more firmly closely contacted with each other. Moreover, force F₁ acts in the second anastomosed part A_{N2} opposite to the side where the mesentery M is located in such a direction as to open the intestinal tracts I_{C1} and I_{C2}, but the force functions so that part where the living tissues are denatured by the discrete electrodes 56b, 58b causes the living tissues to be closely contacted with each other. Therefore, an inter-network is produced between the non-denatured living tissues of the intestinal tracts I_{C1} and I_{C2} such that the living tissues exercise their tissue regeneration power, and the living tissues of the intestinal tracts I_{C1} and I_{C2} are regenerated earlier.

As explained above, according to the embodiment, the following effect can be obtained.

The continuous electrodes 56a, 58a and the discrete electrodes 56b, 58b are arranged on the holding surfaces 62a, 66a of the first and second holding members 52, 54, respectively. Thus, the living tissues (e.g., the intestinal tracts I_{C1} and I_{C2}) between the continuous electrode 56a of the first holding member 52 and the continuous electrode 56a of the second holding member 54 can be heated, denatured and continuously jointed to each other. Consequently, for example, tubular living tissues can be closely contacted with each other or sealed with each other. Moreover, the living tissues (e.g., the intestinal tracts I_{C1} and I_{C2}) between the discrete electrodes 56b of the first holding member 52 and the discrete electrodes 58b of the second holding member 54 can be heated, denatured and jointed to each other. That is, the living tissues can be discretely jointed to each other.

At this time, the part where the living tissues are continuously denatured and jointed to each other is positioned in proximity to the part where the living tissues are discretely denatured and jointed to each other, for example, as shown in FIG. 4B. Further, the living tissues around the part where the living tissues are discretely denatured and jointed to each other are not denatured. Thus, the non-denatured living tissues around the part where the living tissues are discretely denatured and jointed to each other can be kept in contact with (closely contact with) each other. That is, the discrete electrodes 56b, 58b play a major role in maintaining the closely contacted state of the living tissues to which, for example, the force F₁ in a direction to separate them is applied.

For example, when the two intestinal tracts I_{C1} and I_{C2} are anastomosed to each other, the force F₁ acts in such a direction as to separate the intestinal tracts I_{C1} and I_{C2} from each other on the side shown in FIGS. 4A and 4C opposite to the side where the mesentery M is located. However, the intestinal tracts I_{C1} and I_{C2} can be discretely jointed to each other because the intestinal tracts T_{C1} and I_{C2} are discretely jointed to each other by the discrete electrode 56b. Thus, the intestinal tracts I_{C1} and I_{C2} can be kept closely contacted with each other.

Therefore, the parts of the living tissues jointed by the discrete electrodes 56b, 58b serve to draw the living tissues close to each other and keep them closely contacted with each other. That is, the parts of the living tissues jointed by the discrete electrodes 56b, 58b serve to maintain the conglutination of living organisms. Thus, an inter-network is produced between the closely contacted (closed up) living tissues such that the living tissues more easily exercise their tissue regeneration power and can be regenerated earlier.

Although the discrete electrodes 56b of the first holding member 52 are arranged at substantially equal intervals and have about the same area in the embodiment described above, it is also preferable that the distance between the adjacent discrete electrodes 56b varies and that the areas of the discrete electrodes 56b are different from each other. When the tissue is discretely treated with the discrete electrodes 56b, parts contacting the discrete electrodes 56b are denatured, but various modifications of the discrete electrodes 56b are allowed as long as the contact between the living tissues can be maintained without denaturing the part of the living tissue between the discrete electrode 56b and the discrete electrode 56b adjacent thereto.

While the cutter 84 is provided in this embodiment described above, the cutter 84 may not be provided depending on the treatment target. In this case, as described later, the cutter guide groove 94 can function as, for example, a fluid discharge groove (flow channel) for guiding a fluid such as vapor or liquid generated from the living tissues toward the handle 22 of the electro-surgical device 12. Alternatively, the cutter guide groove 94 itself may not be provided.

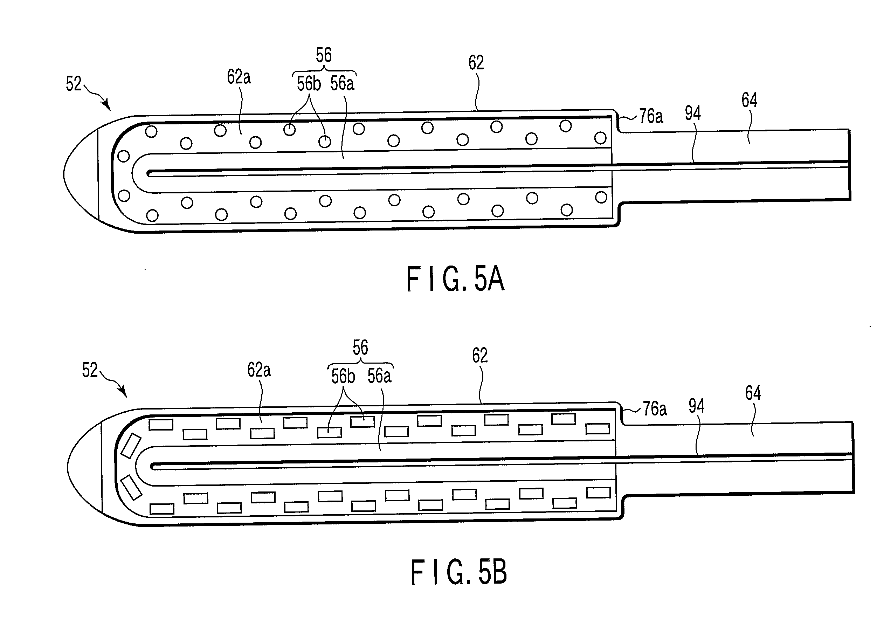

Next, a first modification of the discrete electrode 56b will be explained using FIG. 5A.

In the example of this embodiment described above, the discrete electrodes 56b are arranged at equal intervals on the substantially U-shaped virtual track outside the substantially U-shaped continuous electrode 56a, as shown in FIG. 3A. In addition, it is also preferable that the discrete electrodes 56b are arranged at the apexes of a zigzag shape as shown in FIG. 5A. That is, it is also preferable that the discrete electrodes 56b are arranged in two lines. The arrangement of the discrete electrodes 56b and the distance therebetween in this case are also suitably determined depending on the intensity of the output of the continuous electrode 56a, the areas of the discrete electrodes 56b over the living tissues, etc.

Next, a second modification of the discrete electrodes 56b will be explained using FIG. 5B. The second modification is a further modification of the first modification.

As shown in FIG. 5B, it is also preferable that the rectangular discrete electrodes 56b are arranged in a zigzag form instead of the circular electrodes. In addition, various modifications of the arrangement of the discrete electrodes 56b are allowed, including, for example, a random arrangement. Various modifications of the shape of the discrete electrode 56b are also allowed, including, for example, elliptical, rhomboid and polygonal.

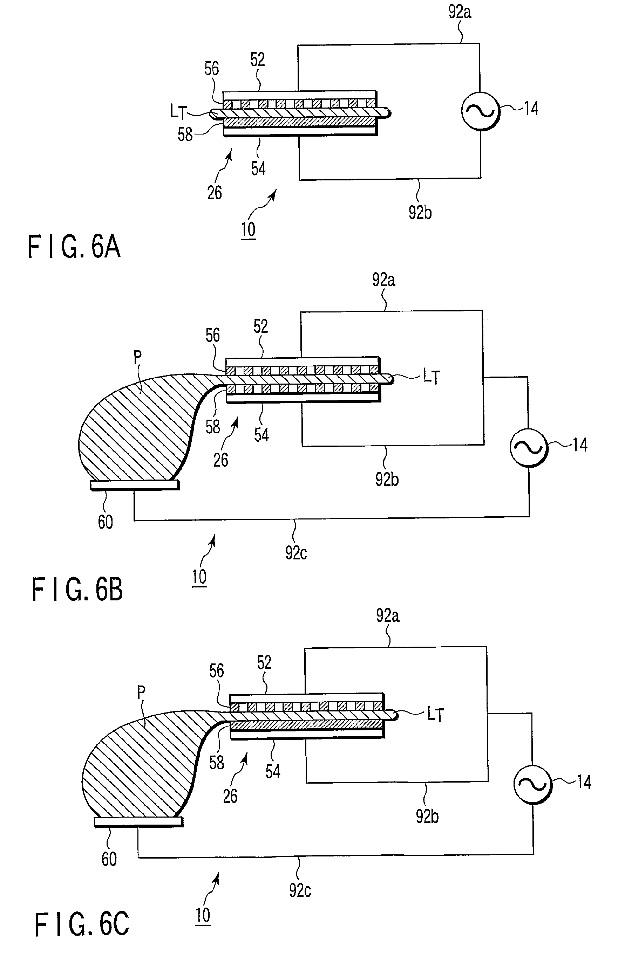

Moreover, the first embodiment has been described using the holding section 26 in which the structure of the main body 62 of the first holding member 52 is symmetrical to (the same as) the structure of the main body 66 of the second holding member 54. In addition, as shown in FIG. 6A, it is also preferable to use the structure described above in the main body 62 of the first holding member 52 and use the entirely exposed planar (plate-like) second high-frequency electrode 58 in the holding surface 66a of the main body 66 of the second holding member 54 on a side proximate to the first holding member 52.

Although using the bipolar-type electro-surgical device 12 has been explained in the embodiment, using a monopolar-type electro-surgical device is also preferable as shown in FIGS. 6B and 6C. In this case, a counter electrode plate 60 is attached to a patient P who is a treatment target. This counter electrode plate 60 is connected with the energy source 14 via an energization line 92c. Further, the first high-frequency electrode 56 arranged on the main body 62 of the first holding member 52 and the second high-frequency electrode 58 arranged on the main body 66 of the second holding member 54 are in the same potential state where the first and second energization lines 92a, 92b are electrically connected with each other. In this case, since an area of the living tissue L_{T} that is in contact with the first and second high-frequency electrodes 56, 58 is small, current density is high, but current density of the counter electrode plate 60 is low. Therefore, the living tissue L_{T} held by the holding section 26 is heated, but the heating of the living tissue L_{T} that is in contact with the counter electrode plate 60 is vanishingly small. Therefore, among the part held by the holding section 26, the living tissue L_{T} being in contact with the electrodes 56, 58 alone is heated and denatured.

Furthermore, although not shown, when the monopolar-type electro-surgical device is used, arranging the high-frequency electrodes on one of the first holding member 52 and the second holding member 54 alone is also preferable.

While using the first and second high-frequency electrodes 56, 58 has been described in the embodiment, it is also preferable to use heaters (not specifically shown because there is no diagrammatic change from, for example, FIG. 3A, FIG. 5A and FIG. 5B) instead of the first and second high-frequency electrodes 56, 58. In addition, it is also preferable to use the continuous electrode 56a as it is and use the heater instead of the discrete electrodes 56b. Still further, it is also preferable to use the discrete electrodes 56b as they are and use the heater instead of the continuous electrode 56a. Thus, when, for example, spotted heaters are used instead of the first and second high-frequency electrodes 56, 58, the heat generation of these heaters enables a treatment to be performed on the living tissue L_{T} in contact with the surfaces of the heaters in the same manner as the treatment performed with the high-frequency electrodes.

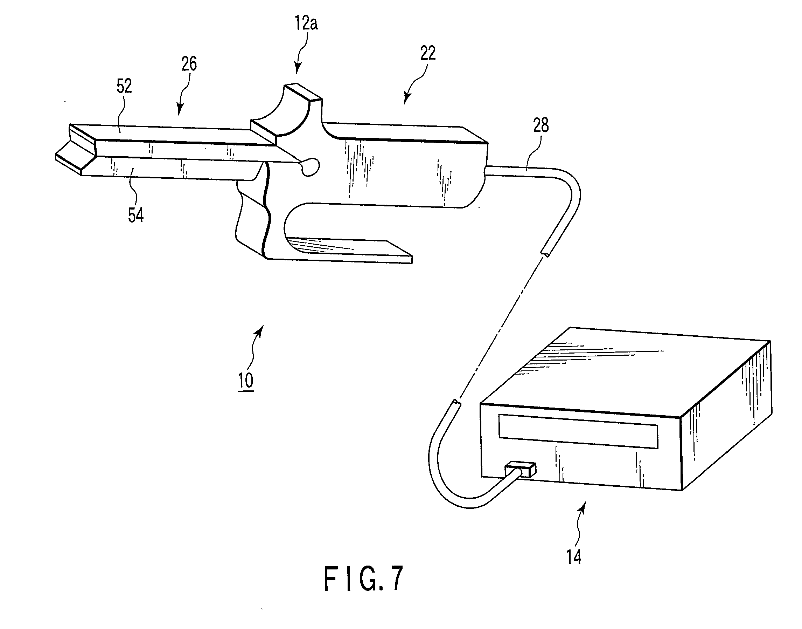

In this embodiment, the linear electro-surgical device 12 (see FIG. 1A) for treating the living tissue L_{T} of the abdominal cavity (in a body) through the abdominal wall has been described as an example. However, for example, as shown in FIG. 7, an open-type linear electro-surgical device (a treatment device for curing) 12a may be used which extracts a treatment target tissue out of the body through the abdominal wall to treat the tissue.

The electro-surgical device 12a includes a handle 22 and a holding section 26. That is, unlike the electro-surgical device 12 for treating the tissue through the abdominal wall, the shaft 24 (see FIG. 1A) is omitted. On the other hand, a member having a function similar to that of the shaft 24 is disposed in the handle 22. Therefore, the device can be used in the same manner as in the electro-surgical device 12 described above with reference to FIG. 1A.

### [Second Embodiment]

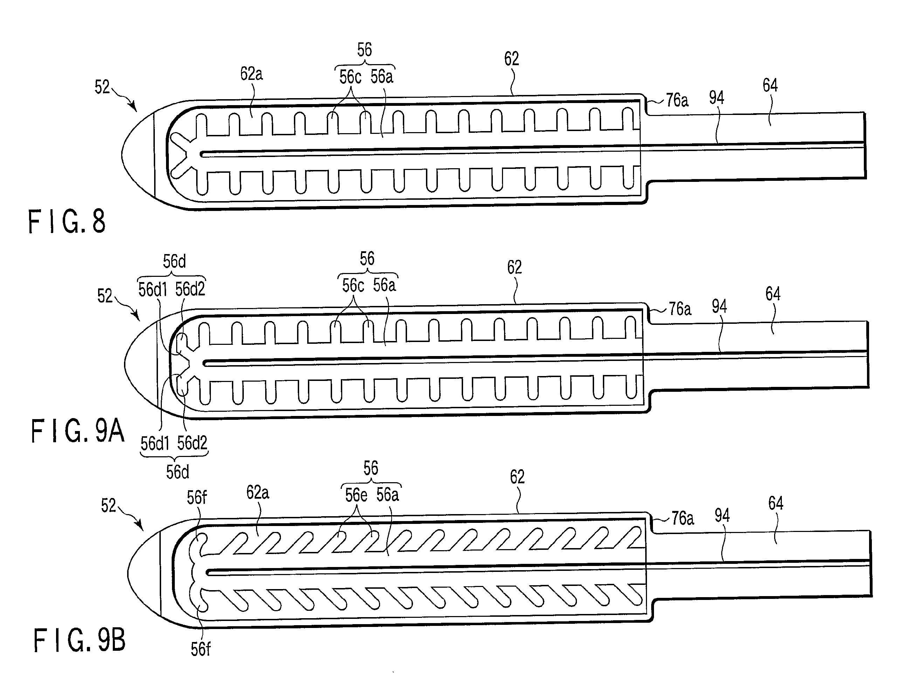

A second embodiment will now be explained with reference to FIGS. 8 to 9B. This embodiment is a modification of the first embodiment, and like reference numbers denote members equal to those explained in the first embodiment, thereby omitting a detailed explanation thereof.

As shown in FIG. 8, a plurality of branched electrodes (maintaining member, second jointing member) branching from a continuous electrode 56a are integrally formed outside the substantially U-shaped continuous electrode 56a. These branched electrodes 56c extend perpendicularly to an axial direction of the continuous electrode 56a. That is, in this embodiment, the branched electrodes 56c are arranged instead of the discrete electrodes 56b described in the first embodiment.

The branched electrodes 56c are formed with about the same length and about the same width. That is, the areas of the branched electrodes 56c extending from the continuous electrode 56a are about the same. Moreover, the distances between the branched electrodes 56c are about the same.

It is to be noted that the branched electrodes 56c denature living tissues L_{T} in contact therewith, but the outputs of the branched electrodes 56c are at such a level that prevents the denaturation of the living tissue L_{T} between the adjacent branched electrodes 56c.

The function and effect of a treatment system 10 according to this embodiment are similar to the function and effect described in the first embodiment and are therefore omitting a description thereof.

It is to be noted that the length and width (thickness) of each of the branched electrodes 56c, the distance between the branched electrodes 56c, and the number of branched electrodes 56c are suitably set. Although the thickness of the continuous electrode 56a is larger than the thickness of the branched electrodes 56c in the illustration of FIG. 8, it is also allowed that the thickness of the continuous electrode 56a is the same as the thickness of the branched electrodes 56c or the thickness of the branched electrodes 56c is larger.

Next, a first modification of the branched electrodes 56c will be explained using FIG. 9A.

As shown in FIG. 9A, branched electrodes (maintaining member, second jointing member) 56d on the most distal side (side distant from a base portion 64) of a main body 62 of a first holding member 52 are modified as compared with the branched electrodes 56c on the most distal side of the main body 62 of the first holding member 52 shown in FIG. 8. That is, the branched electrodes 56d shown in FIG. 9A in the first modification are formed larger than the branched electrodes 56c on the most distal side of the main body 62 of the first holding member 52 shown in FIG. 8.

Furthermore, the branched electrodes 56c on the most distal side shown in FIG. 8 extend only in one direction (straight). In contrast, the angles of the extending branched electrodes 56d shown in FIG. 9A are changed halfway (bent halfway). The purpose is to, when intestinal tracts I_{C1} and I_{C2} as shown in FIG. 4C are anastomosed to each other, increase the force to joint the intestinal tracts I_{C1} and I_{C2} to each other and prevent the release of the anastomosis in the case in which force F₂ acts to release the anastomosis of the intestinal tracts I_{C1} and I_{C2} from the distal end of a part denatured by the continuous electrode 56a, that is, a part Bᵢ where the intestinal tracts I_{C1} and I_{C2} separate in two directions.

The branched electrodes 56d shown in FIG. 9A extend in at least two directions. These branched electrodes 56d include first portions 56d₁ formed integrally with the continuous electrode 56a and extending in a direction perpendicular to the substantially U-shaped virtual track of the continuous electrode 56a, and second portions 56d₂ formed integrally with the first portions 56d₁ and further extending from the first portions 56d₁. Of these portions, the second portions 56d₂ extend in a direction parallel to the branched electrodes 56c. In such a configuration, the branched electrodes 56d have the first portions 56d₁ and the second portions 56d₂ such that it is possible to increase a junction area corresponding to the force F₂ produced at part Bᵢ where the intestinal tracts separate in two directions. That is, the first portions 56d₁ and the second portions 56d₂ prevent the jointed intestinal tracts I_{C1} and I_{C2} from easily separating from each other.

This can increase the resistance to the force F₂ applied to the intestinal tracts I_{C1} and I_{C2}, and thus create a condition where the anastomosis of the intestinal tracts I_{C1} and I_{C2} is not easily released.

Next, a second modification of the branched electrodes 56c will be explained using FIG. 9B.

As shown in FIG. 9B, branched electrodes (maintaining member, second jointing member) 56e of the first holding member 52 are modified as compared with the branched electrodes 56c of the first holding member 52 shown in FIG. 8. The branched electrodes 56e in the second modification are arranged not in a direction perpendicular to but at a slant with respect to the axial direction (substantially U-shaped virtual track) of the continuous electrode 56a. In the case of the second modification, the branched electrodes 56e extend toward, for example, the proximal side.

Thus, as shown in FIG. 4D, the intestinal tracts I_{C1} and I_{C2} include parts jointed by the continuous electrode 56a and parts jointed by the branched electrodes 56e at an angle to the longitudinal direction of the parts jointed by the continuous electrode 56a. Of these electrodes, the branched electrodes 56e are formed longer than the branched electrodes 56c shown in FIG. 8. Moreover, the parts jointed by the branched electrodes 56e are at a slant with respect to the direction of the force F₁ applied to the intestinal tracts I_{C1} and I_{C2}. Therefore, the branched electrodes 56e have increased junction areas corresponding to the force F₁ in a direction to release the anastomosis, such that a condition can be created where the anastomosis of the intestinal tracts I_{C1} and I_{C2} is not easily released. Consequently, the branched electrodes 56e at an angle to the longitudinal direction of the parts connected by the continuous electrode 56a can increase force to joint the intestinal tracts I_{C1} and I_{C2} to each other.

In addition, as shown in FIG. 9B, branched electrodes (maintaining member, second jointing member) 56f on the most distal side of the first holding member 52 are modified as compared with the branched electrodes 56c, 56d on the most distal side of the first holding member 52 shown in FIGS. 8 and 9A. That is, the branched electrodes 56f in the second modification are formed larger than the branched electrodes 56c, 56d on the most distal side of the first holding member 52 shown in FIGS. 8 and 9A.

Furthermore, the branched electrodes 56f shown in FIG. 9B extend in an arc shape. Therefore, the branched electrodes 56f extend in a direction different from the direction of the branched electrodes 56e. The branched electrodes 56f provided on the distal side of the first holding member 52 increase the resistance to the force F₂ produced in the part Bᵢ as shown in FIG. 4C when the intestinal tracts I_{C1} and I_{C2} are anastomosed to each other, thereby making it difficult for the intestinal tracts I_{C1} and I_{C2} to be separated from each other.

The purpose is to, for example, when the intestinal tracts I_{C1} and I_{C2} are anastomosed to each other, increase the force to joint the intestinal tracts I_{C1} and I_{C2} to each other and prevent the release of the anastomosis in the case in which the force F₂ acts to release the anastomosis of the intestinal tracts I_{C1} and I_{C2} from the distal end of a part denatured by the continuous electrode 56a, that is, the part Bᵢ where the intestinal tracts I_{C1} and I_{C2} separate in two directions.

In this embodiment, the branched electrodes 56d having the first portions 56d₁ and the second portions 56d₂, and the branched electrodes 56f have been described as the branched electrodes located on the most distal side of the main body 62 of the first holding member 52 in the case of increasing the area of a junction corresponding to the force F₂. However, the shapes of the branched electrodes located on the most distal side of the main body 62 of the first holding member 52 are not limited to the shapes of the branched electrodes 56d, 56f as long as the area of the junction corresponding to the force F₂ is increased.

### [Third Embodiment]

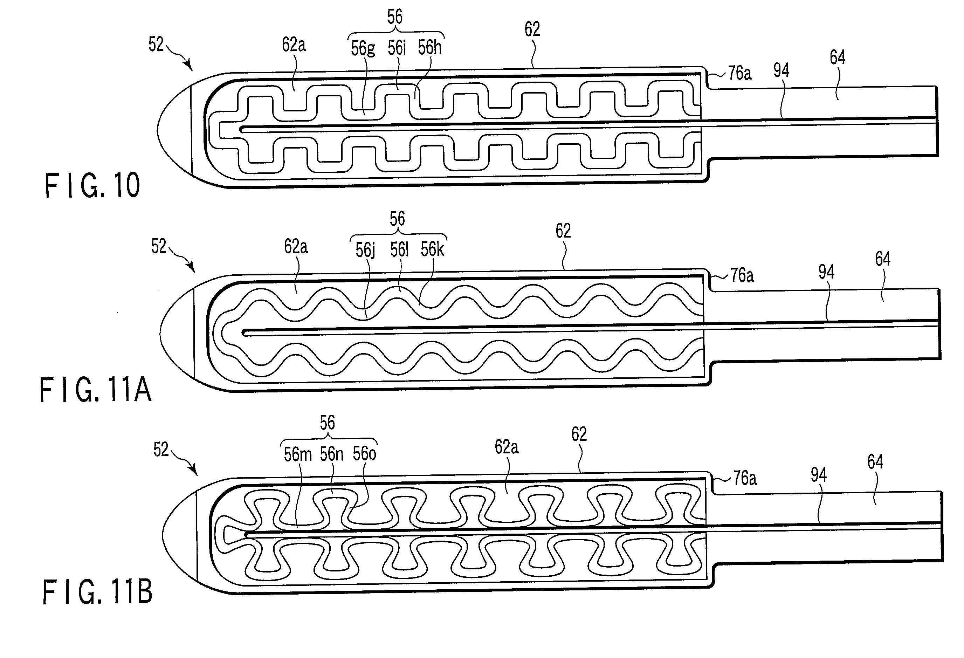

A third embodiment will now be explained with reference to FIGS. 10 to 11B. This embodiment is a modification of the first and second embodiments, and like reference numbers denote members equal to those explained in the first and second embodiments, thereby omitting a detailed explanation thereof.

As shown in FIG. 10, substantially square-wave-shaped continuous high-frequency electrode 56 is disposed in a first holding member 52. Here, electrodes of a part denoted by reference number 56g in FIG. 10 are arranged along an edge of the cutter guide groove 94 in the same manner as the continuous electrode 56a (see FIG. 3A) described in the first and second embodiments. That is, the electrodes 56g are arranged on a substantially U-shaped track. However, the electrodes 56g are discontinuously disposed unlike the continuous electrode 56a.

Electrodes of a part denoted by reference number 56h in FIG. 10 extend about the same length in a direction perpendicular to the electrodes 56g, in the same manner as the branched electrodes 56c (see FIG. 8) described in the second embodiment.

Furthermore, electrodes of a part denoted by reference number 56i in FIG. 10 are arranged to connect the extending ends of the electrodes 56h. Moreover, the electrodes 56i are also discontinuously disposed in the same manner as the electrodes 56g, 56h. That is, the electrodes 56i are arranged separately from each other on the substantially U-shaped track in the same manner as the discrete electrodes 56b (see FIG. 3A) described in the first embodiment.

However, these electrodes 56g, 56h and 56i form one continuous high-frequency electrode 56 as a whole, and therefore have the same effect as the effect of the continuous electrode 56a described in the first and second embodiments.

On the other hand, the electrodes 56h are separate from each other in the same manner as the branched electrodes 56c shown in FIG. 8. Thus, the electrodes 56h serve as parts of the continuous electrode and function as branched electrodes. Moreover, the electrodes 56i serve as parts of the continuous electrode, and discontinuously formed separately from each other and therefore function as discrete electrodes.

That is, these electrodes 56h, 56i are used not only for exerting the function of the continuous electrode to continuously joint the living tissues L_{T} by their denaturation but also for exerting the function of the discrete electrodes 56b shown in FIG. 3A or the branched electrodes 56c shown in FIG. 8 to draw the living tissues L_{T} close to each other and keep them closely contacted with each other.

Although the electrodes denoted by reference number 56g are about the same as the electrodes denoted by reference number 56i in terms of the length and the distance between the electrodes in the illustration of FIG. 10 in this embodiment, the length and distance can be suitably changed. That is, the distance between the electrodes denoted by reference number 56h can be suitably changed.

The function and effect of a treatment system 10 according to this embodiment are similar to the function and effect described in the first and second embodiments and are omitting therefore description thereof.

Next, a first modification of the first high-frequency electrode 56 will be explained using FIG. 11A.

While the substantially square-wave-shaped high-frequency electrode 56 is continuously disposed in the described third embodiment, it is also preferable that this electrode is formed into a substantially sine curve shape as shown in FIG. 11A. When the high-frequency electrode 56 is formed in a substantially sine curve shape as shown in FIG. 11A, electrodes at bottom positions (positions proximate to a cutter guide groove 94) of the substantially sine curve shape denoted by reference number 56j function as replacements of the electrodes 56g described in the third embodiment.

Electrodes at positions of inflection points of the substantially sine curve shape denoted by reference number 56k function as replacements of the electrodes 56h described in the third embodiment. In addition, the electrodes 56k can be regarded as being formed longer than the parts denoted by reference number 56h in FIG. 8. The purpose is to, when, for example, intestinal tracts I_{C1} and I_{C2} are anastomosed to each other as shown in FIG. 4A, prevent the release of the anastomosis, that is, increase the area for jointing the intestinal tracts I_{C1} and I_{C2} to each other in order to increase jointing force, in the case in which the force acts, in a part Bᵢ where the intestinal tracts I_{C1} and I_{C2} separate in two directions, to release the anastomosis of the intestinal tracts I_{C1} and I_{C2} from the distal end of a part denatured by the continuous electrode 56a. Therefore, the electrodes 56k in the first modification have larger areas for contacting the intestinal tracts I_{C1} and I_{C2} than the electrodes 56h shown in FIG. 10, such that the force to joint the intestinal tracts I_{C1} and I_{C2} to each other can be increased.

Similarly, electrodes at top positions (positions distant from the cutter guide groove 94) of the substantially sine curve shape denoted by reference number 561 function as replacements of the electrodes 56i described in the third embodiment.

Next, a second modification of the first high-frequency electrode 56 will be explained using FIG. 11B.

As shown in FIG. 11B, in this modification, electrodes of a part denoted by reference number 56o described in the third embodiment (part corresponding to the branched electrodes 56c shown in FIG. 8) are formed at a slant and longer as compared with the electrodes 56h shown in FIG. 10 described in the third embodiment. The purpose is to, when the intestinal tracts I_{C1} and I_{C2} are anastomosed to each other as shown in FIG. 4A, prevent the release of the anastomosis, that is, increase the area for jointing the intestinal tracts I_{C1} and I_{C2} to each other in order to increase the jointing force in the case in which the force acts, in the part Bᵢ where the intestinal tracts I_{C1} and I_{C2} separate in two directions, to release the anastomosis of the intestinal tracts I_{C1} and I_{C2} from the distal end of a part denatured by the high-frequency electrode 56. Therefore, the electrodes 56o in the second modification have larger areas for contacting the intestinal tracts I_{C1} and I_{C2} than the electrodes 56h shown in FIG. 10, such that the force to joint the intestinal tracts I_{C1} and I_{C2} to each other can be increased.

Furthermore, electrodes of a part denoted by reference number 56m and proximate to a cutter guide groove 94 (at bottom positions of the curve) function as replacements of the electrodes 56g shown in FIG. 10. Electrodes of a part denoted by reference number 56n and distant from the cutter guide groove 94 (at top positions of the curve) function as replacements of the electrodes 56i shown in FIG. 10.

For example, the length of the electrode 56m can be longer than the length of the electrode 56n as in this modification. Naturally, it is also preferable that the length of the electrode 56m is shorter than the length of the electrode 56n.

### [Fourth Embodiment]

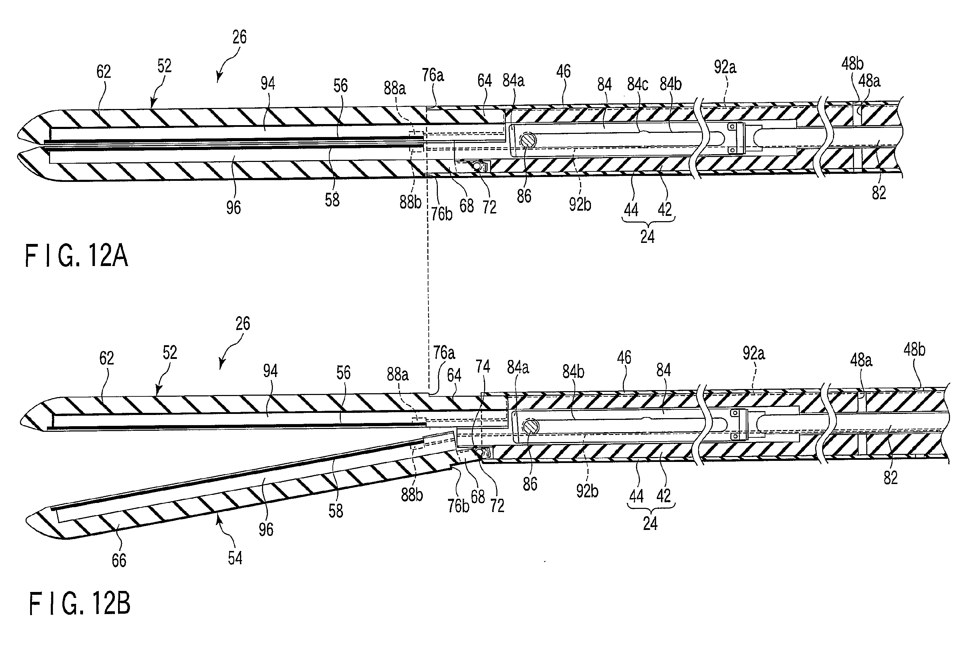

A fourth embodiment will now be explained with reference to FIGS. 12A to 14C. This embodiment is a modification of the first embodiment, and like reference numbers denote members equal to those explained in the first embodiment, thereby omitting a detailed explanation thereof.

In a cylindrical member 42 and a sheath 44 of a shaft 24 of an electro-surgical device 12 shown in FIG. 12A, fluid discharge ports 48a, 48b are formed to discharge a fluid such as vapor (gas) or liquid (tissue fluid) that will be described later. These fluid discharge ports 48a, 48b are formed on the proximal side of the shaft 24.

Although not shown here, it is also preferable that a connection cap is provided on an outer peripheral surface of the fluid discharge port 48b of the sheath 44. At this time, the fluid described later is discharged through a cutter guide groove 94, the fluid discharge port 48a of the cylindrical member 42 of the shaft 24, the fluid discharge port 48b of the sheath 44 of the shaft 24, and the connection cap. In this case, the fluid such as the vapor or liquid can be easily discharged from the fluid discharge ports 48a, 48b by suction through the connection cap.

In addition, it is preferable that the fluid discharge ports 48a, 48b are provided in the shaft 24, but it is also preferable that the fluid discharge ports 48a, 48b are provided in a handle 22 rather than the shaft 24.

The structures of the handle 22 and the shaft 24 are the same as the structures described in the first embodiment in other respects, and other members are therefore omitting an explanation thereof.

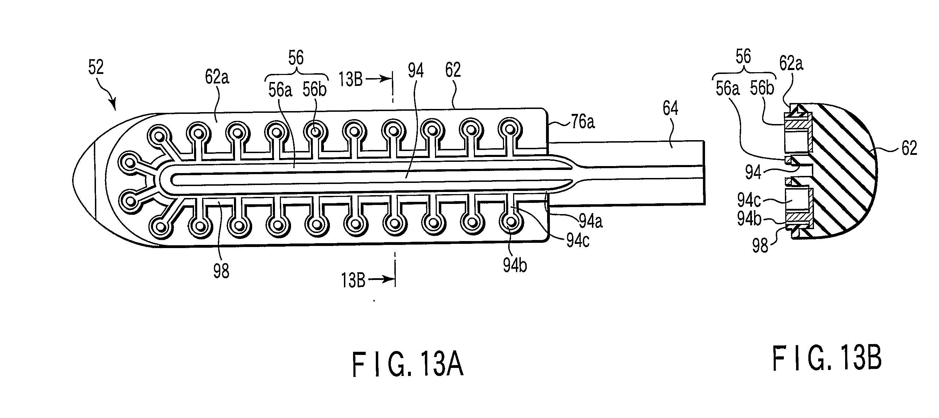

As shown in FIG. 13A, a continuous electrode 56a and discrete electrodes 56b are arranged at about the same positions as those in the first embodiment shown in FIG. 3A.

In a main body 62, a first fluid discharge groove (fluid discharge groove for continuous electrode) 94a is formed on an outer periphery of the continuous electrode 56a as a channel for a fluid such as vapor or high-temperature liquid. In the main body 62, second fluid discharge grooves (fluid discharge grooves for discrete electrode) 94b are also formed on outer peripheries of the discrete electrodes 56b as channels for the fluid such as vapor or high-temperature liquid. The first fluid discharge groove 94a is in communication with the second fluid discharge grooves 94b through communicating passages 94c. Each of the communicating passages 94c is formed as a groove. Moreover, each of the communicating passages 94c is exposed in a holding surface 62a.

As shown in FIGS. 13A and 13B, barrier portions (dams) 98 are formed in the holding surface 62a of a first holding member 52. The barrier portions 98 are formed along the first and second fluid discharge grooves 94a, 94b and edges of the communicating passages 94c. As shown in FIG. 13B, the barrier portions 98 of the first holding member 52 project from a flat surface of the holding surface 62a.

Next, a function of a treatment system 10 according to this embodiment will be described.

As described in the first embodiment, a living tissue L_{T} as a treatment target is held between the first holding member 52 and the second holding member 54. At this time, the barrier portions 98 are closely contacted with the living tissue L_{T}, and the living tissue L_{T} contacts a first high-frequency electrode 56 and a second high-frequency electrode 58.

In this state, a foot switch and a hand switch are operated. An energy source 14 supplies energy to the first high-frequency electrode 56 and the second high-frequency electrode 58. Then, the living tissue L_{T} held between the first high-frequency electrode 56 and the second high-frequency electrode 58 is heated. At this time, for example, a fluid such as vapor or liquid is generated from the heated part of the living tissue L_{T}.

Here, the first fluid discharge groove 94a is disposed outside the continuous electrode 56a, and the second fluid discharge grooves 94b are disposed outside the discrete electrodes 56b. Therefore, the fluid such as the vapor or the liquid flows into the first and second fluid discharge grooves 94a, 94b. At this time, the fluid is prevented from escaping out of the first and second fluid discharge grooves 94a, 94b by the barrier portions 98. That is, the barrier portions 98 serve as the dams for preventing the fluid generated from living tissue L_{T} from leaking out. Therefore, the fluid flown into the second fluid discharge grooves 94b flows into the first fluid discharge groove 94a through the communicating passages 94c. Then, this fluid flows toward a base portion 64 of the first holding member 52 and the second holding member 54. Further, the fluid flows into the cutter guide groove 94 which is in communication with the first fluid discharge groove 94a in, for example, the base portion 64. Alternatively, although not shown, the first fluid discharge groove 94a is in communication therewith inside the cylindrical member 42 of the shaft 24.

Subsequently, the fluid is discharged from the fluid discharge port 48b to the outside of the electro-surgical device 12 through the fluid discharge port 48a of the cylindrical member 42 of the shaft 24.

As explained above, according to this embodiment, the following effects can be obtained.

Effects similar to the effects described in the first embodiment are omitting a description thereof.

When a high-frequency current is applied by the electro-surgical device 12 to the living tissue L_{T} as a treatment target held by the holding section 26, the barrier portions 98 are closely contacted with the living tissue L_{T}, such that the fluid generated from the living tissue L_{T} as a treatment target can be introduced into the first and second fluid discharge grooves 94a, 94b and the communicating passages 94c of the first and second high-frequency electrodes 56, 58 even if the fluid flows toward the barrier portions 98 of the first holding member 52.

In consequence, it can be prevented that the peripheral tissue other than the target tissue is influenced by the fluid generated from the portion to which the high-frequency current has been conducted during the treatment of the living tissue L_{T}. That is, the position influenced during the treatment of the living tissue L_{T} can be limited to the living tissue L_{T} where the high-frequency current has been conducted between the first high-frequency electrode 56 and the second high-frequency electrode 58.

Therefore, according to this embodiment, the fluid such as the vapor or the liquid (high-temperature body fluid) generated from the living tissue L_{T} is discharged to the outside of the electro-surgical device 12, for example, on the proximal side of the shaft 24 or on the side of the handle 22, such that the living tissues L_{T} on the periphery of the living tissue L_{T} as a treatment target can be inhibited from being influenced by the fluid such as the vapor or the liquid (body fluid).

It is important to guide the fluid such as the vapor or the liquid to a position where the fluid does not come into contact with the tissue, when inhibiting a thermal influence on the living tissue L_{T}. It is possible to obtain an especially large effect that the thermal influence can be prevented from being exerted outside the holding section 26, in a case where the tissue is larger than the holding section 26 to such an extent that a surrounding area of the holding section 26 is covered. When an only small opening (space) is made in the holding section 26 but a fluid such as the vapor or the liquid leaks from the opening, the fluid is discharged from the opening, and the living tissue L_{T} around the holding section 26 is thermally influenced.

Furthermore, even if the peripheries of the high-frequency electrodes (energy release portions) 56, 58 are covered with the barrier portions 98 to eliminate such an opening, there is a possibility that an opening is formed by fluid pressure such as vapor pressure generated from the living tissue L_{T} and the fluid is discharged therefrom. Therefore, it is a useful means to provide the flow channels 94a, 94b and 94c which suppress unnecessary discharge of the fluid due to the increase of the fluid pressure and which guide the fluid in a predetermined direction for discharge.

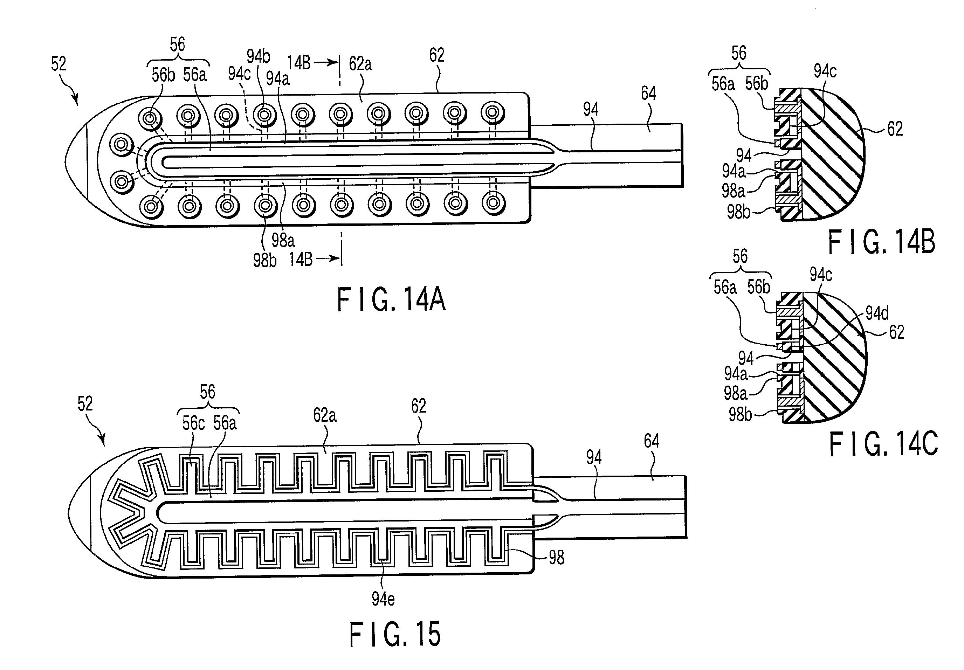

Next, a first modification of the communicating passages 94c shown in FIGS. 13A and 13B will be explained using FIGS. 14A and 14B.

As shown in FIGS. 14A and 14B, the communicating passages (first communicating passages) 94c for communication between the fluid discharge grooves 94a, 94b are formed as conduits. Therefore, each of the communicating passages 94c is formed inside the main body 62.

Thus, the fluid generated from the living tissue L_{T} can be passed through the conduit-shaped communicating passages 94c to maximally prevent the living tissue L_{T} from being touched by the fluid which might be, for example, at a high temperature.

Next, a second modification of the communicating passages 94c shown in FIGS. 13A and 13B will be explained using FIG. 14C. This modification is a further modification of the first modification.

As shown in FIG. 14C, the first communicating passages 94c are formed as conduits as in the first modification. Conduit-shaped communicating passages 94d in communication with the cutter guide groove 94 are formed in the first communicating passages 94c.

Thus, the fluid generated from the living tissue L_{T} can be passed through the first and second conduit-shaped communicating passages 94c, 94d to maximally prevent the living tissue L_{T} from being touched by the fluid which might be, for example, at a high temperature.

### [Fifth Embodiment]

A fifth embodiment will now be explained with reference to FIG. 15. This embodiment is a modification of the second and fourth embodiments, and like reference numbers denote members equal to those explained in the second and fourth embodiments, thereby omitting a detailed explanation thereof.

As shown in FIG. 15, a fluid discharge groove 94e is formed outside a high-frequency electrode 56. Further, a barrier portion 98 is formed at an outer edge of the fluid discharge groove 94e. Here, the barrier portion 98 projects from a holding surface 62a.

The function and effect of a treatment system 10 according to this embodiment are similar to the function and effect described in the fourth embodiment and are omitting an explanation thereof.

### [Sixth Embodiment]

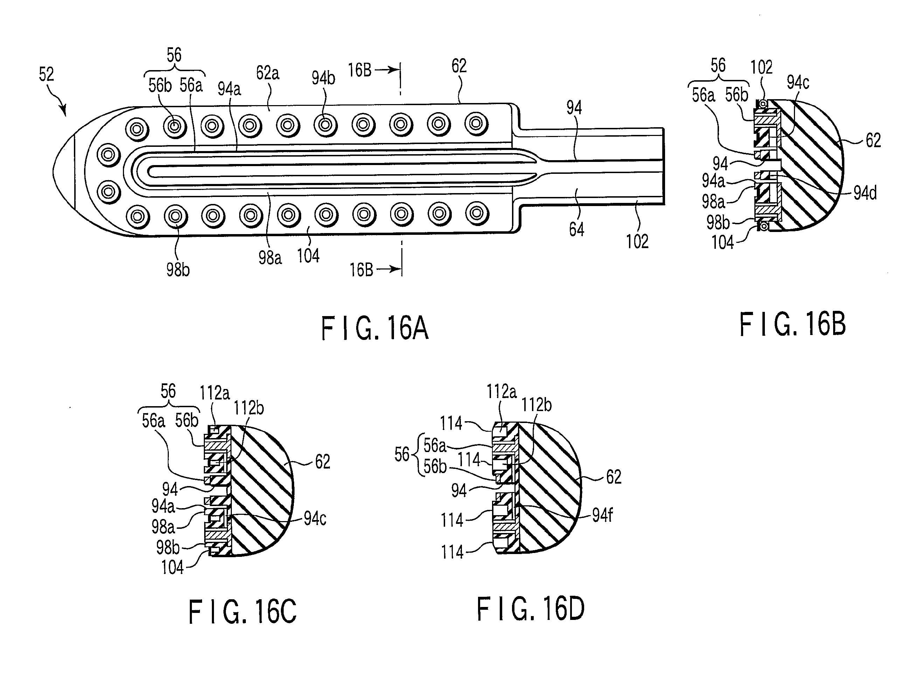

A sixth embodiment will now be explained with reference to FIGS. 16A to 16D. This embodiment is a modification of the first and fourth embodiments, and like reference numbers denote members equal to those explained in the first and fourth embodiments, thereby omitting a detailed explanation thereof.

As shown in FIG. 16B, a communicating passage 94c for communication between vapor discharge grooves 94a, 94b is formed as a conduit, in the same manner as shown in FIG. 14C. Further, a communicating passage 94d for communication between the vapor discharge groove 94a and a cutter guide groove 94 is also formed as a conduit.

As shown in FIGS. 16A and 16B, a cooling pipe 102 made of, for example, copper and having good thermal conductivity is fixed at an edge of a base portion 64 of a main body 62. A cooling medium such as cooling water (liquid) or cool air (gas) is passed through the cooling pipe 102.

A cooling plate 104 made of, for example, a copper plate and having good thermal conductivity is disposed in a holding surface 62a of the main body 62. This cooling plate 104 is fixed to the main body 62 so that it is in close contact with the cooling pipe 102. Therefore, when the cooling medium is passed through the cooling pipe 102, heat from the cooling medium is conducted to the cooling plate 104 from the cooling pipe 102. That is, the cooling plate 104 is cooled off.

A function of a treatment system 10 according to this embodiment will now be explained.

As described in the first embodiment, a living tissue L_{T} as a treatment target is held between first and second holding members 52, 54. At this time, barrier portions 98a, 98b are closely contacted with the living tissue L_{T}, and the living tissue L_{T} contacts first and second high-frequency electrodes 56, 58. Further, the living tissue L_{T} is closely contacted with the cooling plates 104 provided in the main bodies 62, 66 of the first and second holding members 52, 54.

In this state, a foot switch and a hand switch are operated. An energy source 14 supplies energy to the first high-frequency electrode 56 and the second high-frequency electrode 58, respectively. On the other hand, cooling water as a cooling medium is supplied to the cooling pipe 102.

The first high-frequency electrode 56 conducts a high-frequency current between the first high-frequency electrode 56 and the second high-frequency electrode 58 via the living tissue L_{T}. Thus, the living tissue L_{T} between the first high-frequency electrode 56 and the second high-frequency electrode 58 is heated.

Thus, when the living tissue L_{T} as a treatment target is heated, a fluid such as vapor or liquid is generated from the heated part of the living tissue L_{T}. Then, the fluids generated from the living tissue L_{T} meet in the cutter guide groove 94 through the vapor discharge groove 94a provided outside a continuous electrode 56a and through fluid discharge grooves 94b provided outside discrete electrodes 56b, and guided to the proximal side of a shaft 24 and discharged to the outside.

Furthermore, when the living tissue L_{T} as a treatment target is heated, thermal spread is caused from the living tissue L_{T} as a treatment target to the living tissues L_{T} around the treatment target.

Here, the cooling water is supplied to the cooling pipes 102 outside the first and second holding members 52, 54. Thus, the living tissue L_{T} is cooled off via the cooling plates 104 in close contact with the outer peripheral surfaces of the cooling pipes 102 having high thermal conductivity. Therefore, the influence of heat spread from the living tissue L_{T} as a treatment target between the first high-frequency electrode 56 and the second high-frequency electrode 58 is suppressed by parts in close contact with the cooling plates 104. That is, the spread of heat from the living tissue L_{T} as a treatment target is suppressed by cooling off the living tissues L_{T} on the periphery of the living tissue L_{T} as a treatment target.

Moreover, if there are gaps between the living tissues L_{T} and the barrier portions 98a, 98b, the fluid escapes to the outside through the gaps between the living tissues L_{T} and the barrier portions 98a, 98b. In this case, the fluid touches the cooling plates 104. As a result, the fluid is cooled off.

As explained above, according to the embodiment, the following effect can be obtained.

The effects of the treatment system 10 according to this embodiment similar to the effects in the first and fourth embodiments are omitting an explanation thereof.

When a high-frequency current is applied by an electro-surgical device 12 to the living tissue L_{T} as a treatment target held by a holding section 26, the barrier portions 98a, 98b of the first and second holding members 52, 54 can be closely contacted with the living tissue L_{T}. Thus, the fluid generated from the living tissue L_{T} as a treatment target can be introduced into the cutter guide groove 94 through the vapor discharge groove 94a between the continuous electrode 56a and the barrier portion 98a and through the fluid discharge groove 94b between the discrete electrodes 56b and the barrier portion 98b, and discharged to the outside of the electro-surgical device 12, even if the fluid flows toward the barrier portions 98a, 98b of the first and second holding members 52, 54.

In consequence, it can be prevented that the peripheral tissue other than the target tissue is influenced by the fluid generated from the portion to which the high-frequency current has been conducted during the treatment of the living tissue L_{T}. That is, the position influenced during the treatment of the living tissue L_{T} can be limited to the living tissue L_{T} where the high-frequency current has been conducted between the first high-frequency electrode 56 and the second high-frequency electrode 58.

Furthermore, when a high-frequency current is applied by an electro-surgical device 12 to the living tissue L_{T} as a treatment target held by a holding section 26, the cooling plate 104 of the first holding member 52 and the cooling plate 104 of the second holding member 54 that are cooled off can be closely contacted with the living tissue L_{T}. Thus, the living tissue L_{T} closely contacted with the cooling plates 104 can be cooled off. Therefore, the influence of the thermal spread caused from the living tissue L_{T} as a treatment target to the living tissues L_{T} around the treatment target can be suppressed by the portions contacting the cooling plates 104. In consequence, it can be prevented that the peripheral tissue other than the target tissue is influenced by the head spreading from the living tissue L_{T} as a treatment target to which the high-frequency current has been conducted during the treatment of the living tissue L_{T}.

Therefore, the cooling plate 104 capable of cooling off the surface is provided in the holding section 26 such that it is possible to ensure that the thermal spread is caused within the first and second holding members 52, 54.

Still further, even when a high-temperature fluid is to escape out of the first and second holding members 52, 54, this fluid touches the cooling plates 104 and can be cooled off. This can prevent the influence on the living tissues L_{T} on the periphery of the living tissue L_{T} held by the holding section 26.

Moreover, it can be prevented that the peripheral tissue other than the target tissue is influenced by the fluid generated from the portion to which the high-frequency current has been conducted during the treatment of the living tissue L_{T}. That is, the range of the treatment can be restricted to the inside of the barrier portions 98a, 98b, and the living tissue L_{T} around the barrier portions 98a, 98b are kept in a normal condition, which can contribute to earlier curing.

Next, a first modification of this embodiment will be explained using FIG. 16C.

As shown in FIG. 16C, the cooling pipes 102 are eliminated. Instead, groove-like ducts 112a, 112b are integrally formed in the main body 62 and the base portion 64.

The cooling plate 104 is disposed in the holding surface 62a of the main body 62. The ducts 112a, 112b are sealed by this cooling plate 104. Therefore, when a fluid is passed through the ducts 112a, 112b, the heat of this fluid is conducted to the cooling plate 104.

Next, a second modification of this embodiment will be explained using FIG. 16D. This modification is a further modification of the first modification.

As shown in FIG. 16D, the cooling pipes 102 are removed. Instead, the groove-like first and second ducts 112a, 112b are integrally formed in the main body 62 and the base portion 64. A pair of first ducts 112a and a pair of second ducts 112b are formed symmetrically with respect to the central axis of the cutter guide groove 94 of the first holding member 52. One of the pair of each of the first and second ducts 112a, 112b is used for the inflow of a cooling medium such as cooling water, and the other is used for the outflow of the cooling medium.

In addition, a communicating passage 94f for communication between the fluid discharge groove 94a and the cutter guide groove 94 is formed under the second duct 112b.

A thin flexible sheet-like member (heat releasing member) 114 is disposed in the holding surface 62a of the main body 62. This sheet-like member 114 is formed of, for example, a silicone member. The ducts 112a, 112b are sealed by this sheet-like member 114. Therefore, when a fluid is passed through the ducts 112a, 112b, the heat of this fluid is conducted to the living tissue L_{T} through the sheet-like member 114.

### [Seventh Embodiment]

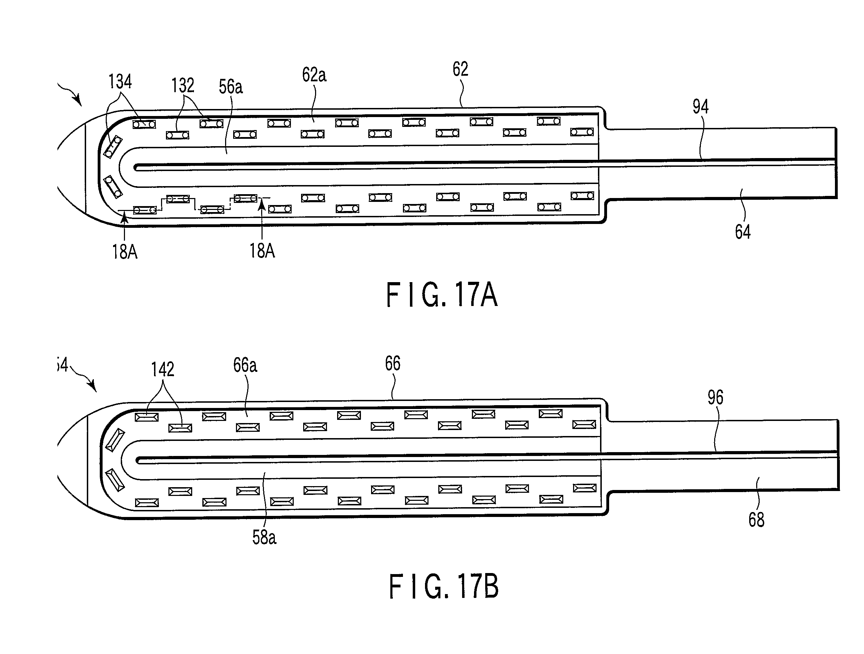

A seventh embodiment will now be explained with reference to FIGS. 17A to 18. This embodiment is a modification of the first embodiment, and like reference numbers denote members equal to those explained in the first embodiment, thereby omitting a detailed explanation thereof.

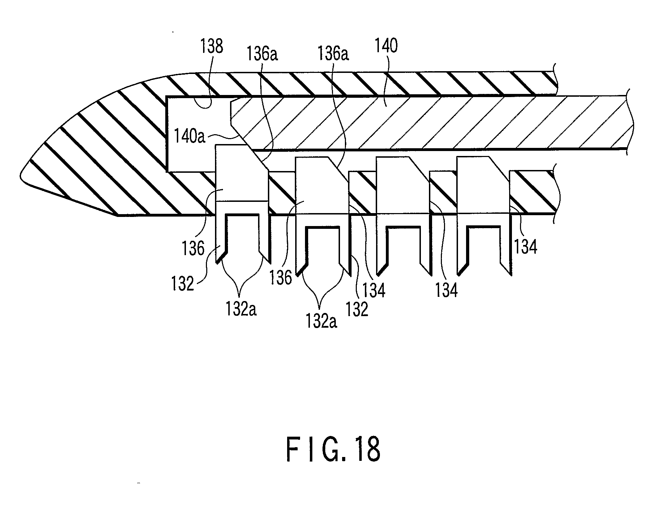

As shown in FIG. 17A, the discrete electrodes 56b (see FIG. 3A) are eliminated from a first holding member 52, and a plurality of slots or slits 134 for discharging surgical staples (maintaining member, second jointing member, applying portion) are formed instead. The plurality of slots 134 are arranged in, for example, two lines on both sides of a cutter guide groove 94 and outside a continuous electrode 56a. As shown in FIG. 18, staple pushers 136 having inclined surfaces 136a are movably housed backward and forward in the slots 134. Staples 132 are housed one by one on the upper surfaces of the staple pushers 136 so that their legs 132a are directed to a second holding member 54 and can project toward a holding surface 66a of the second holding member 54. In the first holding member 52, a plate guide groove 138 is formed opposite to the staple pushers 136. An actuation sled 140 having an inclined surface 140a which is inclined in the same manner as the inclined surfaces 136a of the staple pushers 136 is slidably provided in the plate guide groove 138. The actuation sled 140 is disposed parallel to a cutter 84 and a driving rod 82, and the movement of this actuation sled 140 can be operated, in the same manner as a cutter driving knob 34, by a handle 22 using a staple operating unit (not shown) driven substantially parallel to the cutter 84 and the driving rod 82. Therefore, the first holding member 52 also functions as a cartridge of the staples 132.

As shown in FIG. 17B, the discrete electrodes 58b are eliminated from the second holding member 54, and deforming grooves 142 of the staples 132 are provided instead. That is, a plurality of staple deforming grooves 142 are formed in the holding surface 66a of the second holding member 54. Each of the staple deforming grooves 142 is formed with, for example, a substantially arc-shaped bottom so that the pair of legs 132a (see FIG. 18) of the staple 132 can be folded inwards and deformed. Therefore, the second holding member 54 also functions as an anvil of the staples 132.

It is to be noted that the slots 134 shown in FIG. 17A are formed opposite to the staple deforming grooves 142 shown in FIG. 17B.

That is, this treatment system 10 is equipped with an electro-surgical device 12 for performing a physical treatment using the high-frequency electrodes 58a, 58b and also performing a mechanical treatment using the staples.

Next, a function of the treatment system 10 according to this embodiment will be described.

As described in the first embodiment, a living tissue L_{T} as a treatment target is held between the first holding member 52 and the second holding member 54. At this time, the living tissue L_{T} contacts the continuous electrodes 56a, 58a of first and second high-frequency electrodes 56, 58.

In this state, a foot switch and a hand switch are operated. An energy source 14 supplies energy to the continuous electrodes 56a, 58a, respectively. Then, the living tissue L_{T} between the continuous electrodes 56a, 58a is heated. Thus, the living tissues L_{T} are continuously jointed.

The staple operating unit (not shown) of the handle 22 is operated while the living tissue L_{T} as a treatment target is being held between the first holding member 52 and the second holding member 54. Then, the actuation sled 140 moves forward. Here, as shown in FIG. 18, the actuation sled 140 moves forward along the plate guide groove 138.

When the actuation sled 140 moves forward, the inclined surface 136a of the staple pusher 136 in the slot 134 is pushed down by the inclined surface 140a at the distal end of the actuation sled 140. Thus, the staple 132 in the slot 134 projects and is inserted into the living tissues L_{T} targeted for anastomosis. The pair of legs 132a of the inserted staple 132 penetrates the living tissues L_{T}. Then, the legs 132a are folded inwards by the deforming groove 142 of the second holding member 54. Thus, the tissues targeted for anastomosis are anastomosed to each other by the staple 132. As a result, the living tissues L_{T}, for example, are discretely jointed to each other in a mechanical manner.

As explained above, according to the embodiment, the following effect can be obtained.

The continuous electrodes 56a, 58a are disposed in the holding surfaces 62a, 66a of the first and second holding members 52, 54, respectively. Thus, the living tissues L_{T} (e.g., the intestinal tracts I_{C1} and I_{C2}) between the continuous electrode 56a of the first holding member 52 and the continuous electrode 56a of the second holding member 54 can be heated, denatured and continuously jointed to each other. Consequently, for example, tubular living tissues L_{T} can be closely contacted with each other or sealed with each other.

Furthermore, the staple 132 is discharged from the slot 134 outside the continuous electrode 56a of the first holding member 52, and the pair of legs 132a of the staple 132 is folded inwards in the staple deforming grooves 142 outside the continuous electrode 58a of the second holding member 54. Consequently, the living tissues L_{T} can be discretely jointed mechanically by the staples 132 in the vicinity of the outer side of the continuously jointed part.

At this time, for example, as shown in FIG. 4B, the part where the living tissues L_{T} are continuously denatured and jointed to each other (physically jointed part) is located in proximity to the discretely and mechanically jointed part. Moreover, the living tissues L_{T} around the discretely jointed part are not denatured because they are mechanically jointed to each other by the staple 132. Thus, the non-denatured living tissues L_{T} around the discretely jointed part can be kept pressed (in contact with) against each other. That is, the staple 132 plays a major role in maintaining the closely contacted state of the living tissues L_{T} to which, for example, force in a direction to separate them is applied.

For example, when the two intestinal tracts I_{C1} and I_{C2} are anastomosed to each other, force acts in such a direction as to separate the intestinal tracts I_{C1} and I_{C2} from each other on the side shown in FIGS. 4A and 4C opposite to the side where a mesentery M is located. However, the intestinal tracts I_{C1} and I_{C2} can be discretely jointed to each other because the intestinal tracts I_{C1} and I_{C2} are discretely jointed to each other by the staples 132. Thus, the intestinal tracts I_{C1} and I_{C2} can be kept closely contacted with each other.

Therefore, the parts of the living tissues L_{T} jointed by the staples 132 serve to draw the living tissues L_{T} close to each other and keep them closely contacted with each other. Thus, an inter-network is produced between the closely contacted living tissues L_{T} such that the living tissues L_{T} more easily exercise their tissue regeneration power and can be regenerated earlier.

Although the staples 132 are used to mechanically joint the living tissues L_{T} to each other in the example explained in this embodiment, it is also preferable that, for example, a living tissue adhesive is used to joint the living tissues L_{T} to each other.

### [Eighth Embodiment]

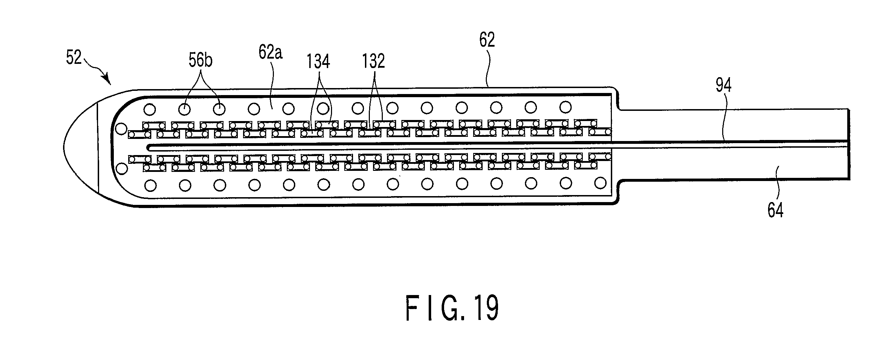

An eighth embodiment will now be explained with reference to FIG. 19. This embodiment is a modification of the first and seventh embodiments, and like reference numbers denote members equal to those explained in the first and seventh embodiments, thereby omitting a detailed explanation thereof.

As shown in FIG. 19, the continuous electrode 56a is eliminated from a first holding member 52 as compared with the first embodiment (see FIG. 3A), and slots 134 for discharging staples 132 are disposed instead. That is, a plurality of slots 134 in which the staples (sealing member, applying portions, first jointing member) 132 are disposed are formed in a holding surface 62a of the first holding member 52. Discrete electrodes 56b of, for example, circular shapes are disposed without change.

The slots 134 for discharging the staples 132 are formed in, for example, two lines along each of the two sides of a cutter guide groove 94 in a closely spaced and dense manner. That is, the arrangement of the discrete electrodes 56b, 58b, the slots 134 of the staples 132 and staple deforming grooves 142 in this embodiment is opposite to the arrangement of the continuous electrodes 56a, 58a, the slots 134 of the staples 132 and the staple deforming grooves 142 described in the seventh embodiment (see FIGS. 17A and 17B).

A function of a treatment system 10 according to this embodiment will now be explained.

As described in the first embodiment, a living tissue L_{T} as a treatment target is held between the first holding member 52 and a second holding member 54. At this time, the living tissue L_{T} contacts the discrete electrodes 56b, 58b of the first and second high-frequency electrodes 56, 58.

In this state, a staple operating unit (not shown) of a handle 22 is operated. Then, as described in the seventh embodiment, the staple 132 in the slot 134 projects and is inserted into the living tissues L_{T} targeted for anastomosis. A pair of legs 132a of the inserted staple 132 penetrates the living tissues L_{T}. Then, the legs 132a are folded inwards by the deforming groove 142 positioned in proximity to a cutter guide groove 96 of the second holding member 54. Thus, the tissues L_{T} targeted for anastomosis are anastomosed to each other by the staples 132. As a result, the living tissues L_{T} are mechanically jointed to each other in a closely spaced and continuous manner.

In this state, a foot switch and a hand switch are operated. An energy source 14 supplies energy to the discrete electrodes 56b, 58b, respectively. Then, the living tissue L_{T} between the discrete electrodes 56b, 58b is heated, denatured and jointed.

As explained above, according to the embodiment, the following effect can be obtained.

The staple 132 is discharged from the slot 134 inside the discrete electrodes 56b of the first holding member 52, and the pair of legs 132a of the staple 132 is folded inwards in the staple deforming grooves 142 inside the discrete electrodes 58b of the second holding member 54. Consequently, the living tissues L_{T} can be mechanically jointed to each other by the staples 132 in a closely spaced and continuous manner.

Furthermore, the discrete electrodes 56b, 58b are disposed in the holding surfaces 62a, 66a of the first and second holding members 52, 54, respectively. Thus, the living tissues L_{T} (e.g., the intestinal tracts I_{C1} and I_{C2}) between the discrete electrodes 56b of the first holding member 52 and the discrete electrodes 58b of the second holding member 54 can be heated, denatured, and discretely jointed to each other.

Therefore, the living tissues L_{T} can be continuously jointed (sealed) to each other by the staples 132, and the living tissues L_{T} can be discretely jointed to each other by the discrete electrodes 56b, 58b. Thus, according to the eighth embodiment, the effects similar to the effects described in the seventh embodiment can be obtained.

### [Ninth Embodiment]

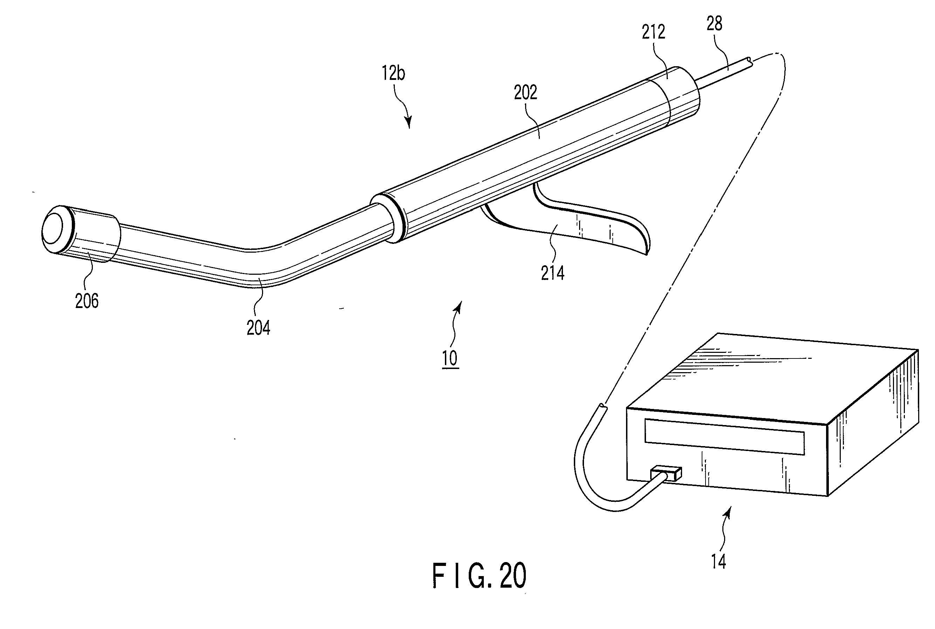

A ninth embodiment will now be explained with reference to FIGS. 20 to 21D. This embodiment is a modification of the first embodiment.

Here, as an example of an energy treatment device, a circular-type bipolar electro-surgical device (a treatment device for curing) 12b will be described which performs a treatment, for example, through an abdominal wall or outside the abdominal wall.

As shown in FIG. 20, the electro-surgical device 12b includes a handle 202, a shaft 204 and an openable/closable holding section 206. The handle 202 is connected with an energy source 14 via a cable 28.

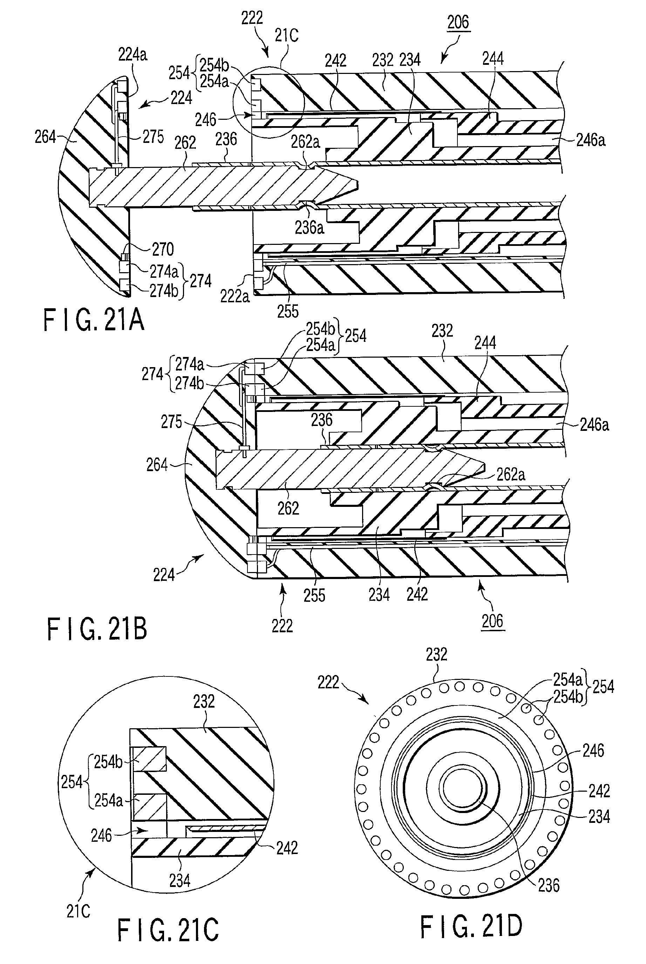

The handle 202 is provided with a holding section opening/closing knob 212 and a cutter driving lever 214. The holding section opening/closing knob 212 is rotatable with respect to the handle 202. When the holding section opening/closing knob 212 is rotated, for example, clockwise with respect to the handle 202, a detachable side holding portion (a detachable side grasping portion) 224 of the holding section 206 described later comes away from a main body side holding portion (a main body side grasping portion) 222 (see FIG. 21A). When the knob 212 is rotated counterclockwise, the detachable side holding portion 224 comes close to the main body side holding portion 222 (see FIG. 21B).

The shaft 204 is formed into a cylindrical shape. This shaft 204 is appropriately curved in consideration of insertability into a living tissue. Needless to say, the shaft 204 may be linearly formed.

A distal end of the shaft 204 is provided with the holding section 206. As shown in FIGS. 21A and 21B, the holding section 206 includes the main body side holding portion (a first holding member) 222 formed at the distal end of the shaft 204, and the detachable side holding portion (a second holding member) 224 detachably attached to the main body side holding portion 222. In a state where the detachable side holding portion 224 is closed with respect to the main body side holding portion 222, holding surfaces 222a, 224a of the main body side holding portion 222 and the detachable side holding portion 224 are opposed to each other.

The main body side holding portion 222 includes a cylindrical member 232, a frame 234 and an electro-conductive pipe 236. The cylindrical member 232 and the frame 234 have an insulating property. The cylindrical member 232 is connected with the distal end of the shaft 204. The frame 234 is fixed to the cylindrical member 232.

A central axis of the frame 234 is opened. The opened central axis of the frame 234 is provided with the electro-conductive pipe 236 which is movable in a predetermined region along the central axis of the frame 234. When the holding section opening/closing knob 212 is rotated, as shown in FIGS. 21A and 21B, the electro-conductive pipe 236 is movable in a predetermined region owing to, for example, a function of a ball screw (not shown). The electro-conductive pipe 236 is provided with a protrusion 236a which protrudes inwards in a diametric direction so that a connecting portion 262a of an electro-conductive shaft 262 described later disengageably engages with the protrusion 236a.

As shown in FIGS. 21A and 21B, a cutter guide groove (a space) 246 is formed between the cylindrical member 232 and the frame 234. A cylindrical cutter 242 is disposed in the cutter guide groove 246 between the cylindrical member 232 and the frame 234. A proximal end of the cutter 242 is connected to a distal end of a pusher 244 for the cutter disposed in the shaft 204. The cutter 242 is fixed to an outer peripheral surface of the pusher 244 for the cutter. Although not shown, a proximal end of the pusher 244 for the cutter is connected to the cutter driving lever 214 of the handle 202. Therefore, when the cutter driving lever 214 of the handle 202 is operated, the cutter 242 moves via the pusher 244 for the cutter 242.

A first fluid flow path (a fluid passage) 246a is formed between the pusher 244 for the cutter and the frame 234. Moreover, the shaft 204 or the handle 202 is provided with a fluid discharge port (not shown) from which the fluid passed through the first fluid flow path 246a is discharged to the outside.

As shown in FIGS. 21A to 21C, a first high-frequency electrode 254 is disposed as an applying portion or an energy emitting portion at the distal end of the cylindrical member 232. The first high-frequency electrode 254 is provided between an edge of the cutter guide groove 246 in which the cutter 242 is disposed and an edge of the cylindrical member 232. A distal end of a first conducting line 255 is fixed to the first high-frequency electrode 254. The first conducting line 255 is connected to the cable 28 via the main body side holding portion 222, the shaft 204 and the handle 202.

As shown in FIG. 21C, the first high-frequency electrode 254 includes a seamlessly and continuously formed continuous electrode (sealing member, first jointing member) 254a, and a plurality of discrete electrodes (maintaining member, second jointing member) 254b discretely disposed outside the continuous electrode 254a.

The continuous electrode 254a is continuously disposed on an outer edge of the cutter guide groove 246, for example, in a substantially annular shape. The discrete electrodes 254b of about the same shape are arranged at substantially equal intervals along a substantially annular virtual track. Each of the discrete electrodes 254b is formed into, for example, a circular shape. The discrete electrodes 254b are arranged to have a predetermined space therebetween, and each of the discrete electrodes 254b is disposed at a distance from the continuous electrode 254a. The discrete electrodes 254b are positioned to, when a treatment is performed, maximally prevent the denaturation of a living tissue L_{T} between the adjacent discrete electrodes 254b due to heat and also maximally prevent the denaturation of a living tissue L_{T} between the discrete electrode 254b and the continuous electrode 254a due to heat.

On the other hand, the detachable side holding portion 224 includes an energization shaft 262 having a connecting portion 262a, and a head portion 264. The energization shaft 262 has a circular cross section, one end formed into a tapered shape, and the other end being fixed to the head portion 264. The connecting portion 262a is formed into a concave groove shape allowing engagement with a protrusion 236a of the energization pipe 236. An outer surface of the energization shaft 262 except the connecting portion 262a is insulated by using, e.g., a coating.

A cutter receiving portion 270 having an annular shape is provided in the head portion 264. A second high-frequency electrode 274 as an output member or an energy applying portion is provided outside the cutter receiving portion 270. The second high-frequency electrode 274 is provided between the cutter receiving portion 270 and an edge of the head portion 264. One end of a second energization line 275 is fixed to the second high-frequency electrode 274. The other end of the second energization line 275 is electrically connected with the energization shaft 262.

In addition, the second high-frequency electrode 274 includes a seamlessly and continuously formed continuous electrode (sealing member, first jointing member) 274a, and a plurality of discrete electrodes (maintaining member, second jointing member) 274b discretely disposed outside the continuous electrode 274a. The continuous electrode 274a is positioned opposite to the continuous electrode 254a of the main body side holding portion 222, and each of the discrete electrodes 274b is positioned opposite to each of the discrete electrodes 254b of the main body side holding portion 222. That is, the continuous electrode 274a is continuously disposed outside the cutter receiving portion 270, for example, in a substantially annular shape. The discrete electrodes 274b of substantially the same shape are arranged at substantially equal intervals along a substantially annular virtual track. Each of the discrete electrodes 274b is formed into, for example, a circular shape. The discrete electrodes 274b are arranged to have a predetermined space therebetween, and each of the discrete electrodes 274b is disposed at a distance from the continuous electrode 274a. The discrete electrodes 274b are positioned to maximally prevent the denaturation of a living tissue L_{T} between the adjacent discrete electrodes 274b due to heat and also maximally prevent the denaturation of a living tissue L_{T} between the discrete electrode 274b and the continuous electrode 274a due to heat.

It is to be noted that the electro-conductive pipe 236 is connected with the cable 28 via the shaft 204 and the handle 202. Therefore, when the connecting portion 262a of the electro-conductive shaft 262 of the detachable side holding portion 224 is engaged with the protrusion 236a of the electro-conductive pipe 236, the second high-frequency electrode 274 is electrically connected with the electro-conductive pipe 236.

Next, a function of a treatment system 10 according to this embodiment will be described.

As shown in FIG. 21B, in a state in which the main body side holding portion 222 is closed with respect to the detachable side holding portion 224, the holding section 206 and the shaft 204 of the electro-surgical device 12b are inserted into an abdominal cavity through, for example, an abdominal wall. The main body side holding portion 222 and the detachable side holding portion 224 of the electro-surgical device 12b is opposed to the living tissue to be treated.

The holding section opening/closing knob 212 of the handle 202 is operated in order to hold the living tissue to be treated between the main body side holding portion 222 and the detachable side holding portion 224. At this time, the knob 212 is rotated, for example, clockwise with respect to the handle 202. In this case, as shown in FIG. 21A, the electro-conductive pipe 236 is moved toward a distal end with respect to the frame 234 of the shaft 204. Therefore, the main body side holding portion 222 and the detachable side holding portion 224 are opened, and the detachable side holding portion 224 can be detached from the main body side holding portion 222.

Moreover, the living tissue to be treated is disposed between the first high-frequency electrode 254 of the main body side holding portion 222 and the second high-frequency electrode 274 of the detachable side holding portion 224. The electro-conductive shaft 262 of the detachable side holding portion 224 is inserted into the electro-conductive pipe 236 of the main body side holding portion 222. In this state, the holding section opening/closing knob 212 of the handle 202 is rotated, for example, counterclockwise. Therefore, the detachable side holding portion 224 closes with respect to the main body side holding portion 222. In this manner, the living tissue of the treatment target is held between the main body side holding portion 222 and the detachable side holding portion 224.

In this state, a foot switch and a hand switch are operated. The energy source 14 supplies energy to the first high-frequency electrode 254 and the second high-frequency electrode 274 via the cable 28, respectively. The continuous electrode 254a and the discrete electrodes 254b of the first high-frequency electrode 254 conduct a high-frequency current to the continuous electrode 274a and the discrete electrodes 274b of the second high-frequency electrode 274 via the living tissue. Thus, the living tissue between the main body side holding portion 222 and the detachable side holding portion 224 is heated.

Then, the living tissue L_{T} is continuously (substantially annularly) denatured by the continuous electrode 254a and the continuous electrode 274a of the first and second high-frequency electrodes 254, 274. Further, the living tissues L_{T} around the part denatured by the continuous electrode 254a of the first high-frequency electrode 254 and the continuous electrode 274a of the second high-frequency electrode 274 are discretely denatured by the discrete electrodes 254b, 274b of the first and second high-frequency electrodes 254, 274.

When a treatment is finished, the operation of the foot switch or the hand switch is stopped. Then, the supply of energy from the energy source 14 to the first high-frequency electrode 254 and the second high-frequency electrode 274 is stopped.

Additionally, when the cutter driving lever 214 of the handle 202 is operated, the cutter 242 protrudes from the cutter guide groove 246 of the main body side holding section 222 and moves toward a cutter receiving portion 270 of the detachable side holding portion 224. Since the cutter 242 has a blade at a distal end thereof, the treated living tissue is cut into a circular shape.

As explained above, according to this embodiment, the following effects can be obtained.

The continuous electrodes 254a, 274a and the discrete electrodes 254b, 274b are disposed in the main body side holding section 222 and the detachable side holding portion 224. Thus, the living tissue L_{T} between the continuous electrode 254a of the main body side holding section 222 and the continuous electrode 274a of the detachable side holding portion 224 can be heated, denatured, and jointed to each other. Therefore, the living tissues L_{T} can be substantially annularly sealed with each other. Moreover, the living tissue L_{T} between the discrete electrodes 254b of the main body side holding section 222 and the discrete electrodes 274b of the detachable side holding portion 224 can be heated, denatured, and jointed to each other. As a result, the living tissues L_{T} can be discretely jointed to each other.

At this time, the part where the living tissues L_{T} are continuously denatured and jointed to each other is located in proximity to the discretely jointed part. Moreover, the living tissues L_{T} around the discretely denatured and jointed part are not denatured. Thus, the non-denatured living tissues L_{T} around the discretely denatured and jointed part can be in contact with (pressed against) each other. Therefore, the discrete electrodes 254b, 274b play a major role in maintaining the closely contacted state of the living tissues L_{T} to which, for example, force in a direction to separate them is applied. Therefore, the parts of the living tissues L_{T} jointed by the discrete electrodes 254b, 274b serve to draw the living tissues L_{T} close to each other. Thus, an inter-network is produced between the living tissues L_{T} such that the living tissues L_{T} more easily exercise their tissue regeneration power and can be regenerated earlier.

Although the discrete electrodes 254b of the main body side holding section 222 are arranged at equal intervals and have a substantially equal area in the embodiment described above, it is also preferable that the areas of the discrete electrodes 254b are different from each other and that the distance between the adjacent discrete electrodes 254b varies. In this case, when the tissue is discretely treated with the discrete electrodes 254b, a part contacting the discrete electrode 254b is denatured, but various modifications of the discrete electrodes 254b are allowed as long as the living tissues L_{T} can be left in contact with each other without denaturing the living tissues L_{T} around the denatured part. This holds true with the detachable side holding portion 224.

Although using the bipolar-type electro-surgical device 12b has been explained in the embodiment, using a monopolar-type electro-surgical device is also preferable as shown in FIGS. 6B and 6C.

While using the first and second high-frequency electrodes 254, 274 has been described in the embodiment, it is also preferable to use heaters (not specifically shown because there is no diagrammatic change from, for example, FIG. 21A to FIG. 21D) instead of the first and second high-frequency electrodes 254, 274. In addition, it is also preferable to use the continuous electrodes 254a, 274a as they are and use the heater instead of the discrete electrodes 254b, 274b. Still further, it is also preferable to use the discrete electrodes 254b, 274b as they are and use the heater instead of the continuous electrodes 254a, 274a. Thus, when, for example, spotted heaters are used instead of the first and second high-frequency electrodes 254, 274, the heat generation of these heaters enables a treatment to be performed on the living tissue L_{T} in contact with the surfaces of the heaters in the same manner as the treatment performed with the high-frequency electrodes.

### [Tenth Embodiment]

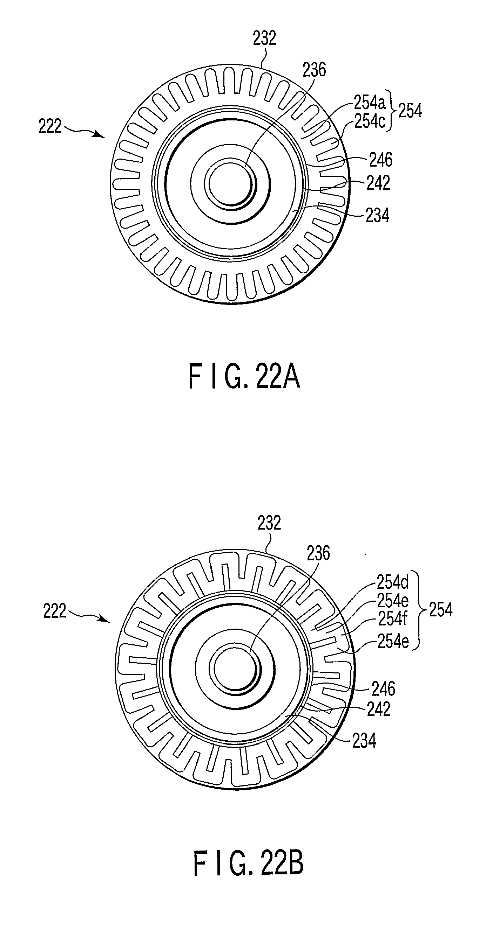

A tenth embodiment will now be explained with reference to FIG. 22A. This embodiment is a modification of the second, third and ninth embodiments.

As shown in FIG. 22A, branched electrodes (maintaining member, second jointing member) 254c are integrally formed outside a continuous electrode 254a. The branched electrodes 254c extend perpendicularly (diametrically outwardly) to an axial direction (circumferential direction) of the continuous electrode 254a. That is, in this embodiment, the branched electrodes 254c are arranged instead of the discrete electrodes 254b described in the eighth embodiment.

The branched electrodes 254c are formed with about the same length and about the same width. That is, the areas of the branched electrodes 254c extending from the continuous electrode 254a are about the same. Moreover, the distances between the branched electrodes 254c are about the same.

It is to be noted that the adjacent branched electrodes 254c denature living tissue in contact therewith, but the outputs of the branched electrodes 254c are at such a level that prevents the denaturation of the living tissue between the adjacent branched electrodes 254c.

The function and effect of a treatment system 10 according to this embodiment are similar to the function and effect described in the ninth embodiment and are omitting an explanation thereof.

### [Eleventh Embodiment]

An eleventh embodiment will now be explained with reference to FIG. 22B. This embodiment is a modification of the ninth and tenth embodiments.

As shown in FIG. 22B, a continuous high-frequency electrodes 254 of a substantially sine curve shape is disposed in a main body side holding section 222. Here, electrodes of a part denoted by reference number 254d in FIG. 22B are arranged along an outer edge of a cutter guide groove 94 in the same manner as the continuous electrode 254a described in the ninth and tenth embodiments. However, the electrodes 254d are discontinuously disposed unlike the continuous electrode 254a. Electrodes of a part denoted by reference number 254d in FIG. 22B extend about the same length in a direction perpendicular to the electrodes 254d, in the same manner as the branched electrodes 254c (see FIG. 22A) described in the tenth embodiment. Further, electrodes of a part denoted by reference number 254f in FIG. 22B are arranged to connect the extending ends of the electrodes 254e. Moreover, the electrodes 254f are also discontinuously disposed in the same manner as the electrodes 254d.

However, these electrodes 254d, 254e and 254f form one continuous high-frequency electrode 254 as a whole, and therefore have the same effects as the effects of the continuous electrode 254a described in the ninth and tenth embodiments.

The electrodes 254d serve as parts of the continuous electrode, and discontinuously formed separately from each other and therefore function as discrete electrodes. The electrodes 254e are separated from each other in the same manner as the branched electrodes 254c shown in FIG. 22A. Thus, the electrodes 254e serve as parts of the continuous electrode and function as branched electrodes. Moreover, the electrodes 254f serve as parts of the continuous electrode, and discontinuously formed separately from each other and therefore function as discrete electrodes.

That is, these electrodes 254d, 254e and 254f are used not only for exerting the function of the continuous electrode to continuously joint the living tissues through their denaturation but also for exerting the functions similar to those of the discrete electrodes 254b shown in FIG. 21D and the branched electrodes 254c shown in FIG. 22A to keep them closely contacted with (pulled close to) each other.

Although the length and the distance between the electrodes are about the same in the electrodes denoted by reference number 254d, the electrodes denoted by reference number 254e and the electrodes denoted by reference number 254f in the illustration of FIG. 22B in this embodiment, the length and distance can be suitably changed.

The effects of a treatment system 10 according to this embodiment are similar to the effects in the ninth and tenth embodiments and are therefore omitting a description thereof.

Therefore, various modifications of the electrode 254 are allowed as in, for example, FIGS. 11A and 11B showing the modifications of the third embodiment.

### [Twelfth Embodiment]

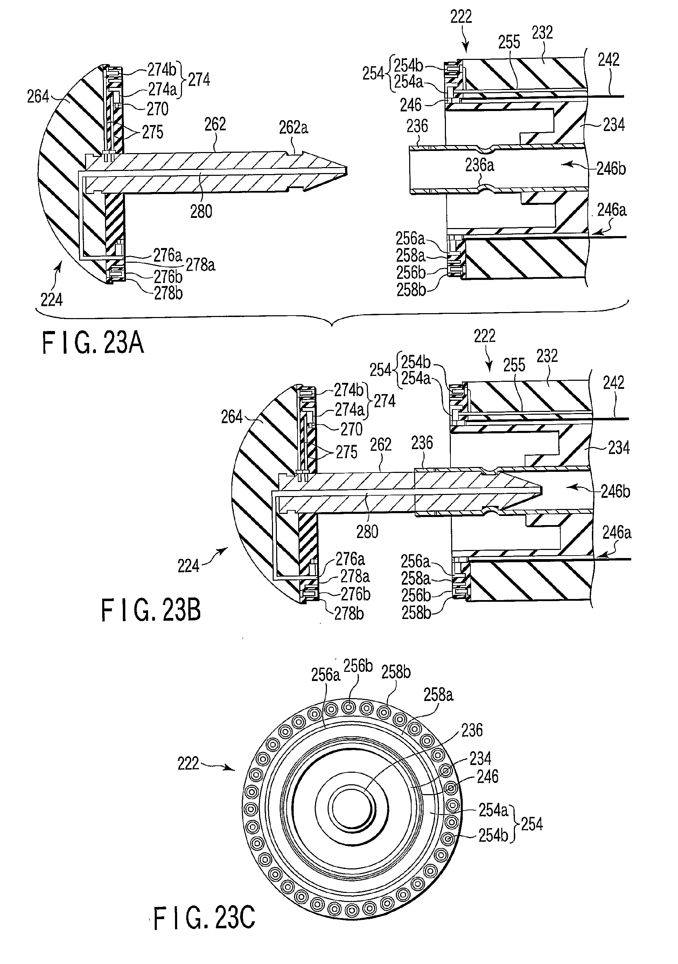

A twelfth embodiment will now be explained with reference to FIGS. 23A to 23C. This embodiment is a modification of the fourth to sixth, and ninth to eleventh embodiments.

As shown in FIGS. 23A to 23C, a vapor discharge groove 256a is annularly formed outside a continuous electrode 254a of a first high-frequency electrode 254. This fluid discharge groove 256a is in communication with a first fluid flow path 246a in which a cutter 242 is disposed. A barrier portion 258a is formed outside the fluid discharge groove 256a at a position higher than the surface of the continuous electrode 254a. That is, the barrier portion 258a of a main body side holding section 222 is closer to a head portion 264 of a detachable side holding portion 224 than the surface of the continuous electrode 254a.

A vapor discharge groove 256b is annularly formed outside a discrete electrode 254b of the first high-frequency electrode 254. This fluid discharge groove 256b is in communication with the fluid discharge groove 256a and the first fluid flow path 246a. A barrier portion 258b is formed outside the fluid discharge groove 256b at a position higher than the surface of the vapor discharge groove 256b. That is, the barrier portion 258b of the main body side holding section 222 is closer to the head portion 264 of the detachable side holding portion 224 than the surface of the first high-frequency electrode 254.

It is to be noted that the surfaces of the barrier portions 258a, 258b are substantially flush with each other.

A fluid discharge groove 276a is annularly formed outside a continuous electrode 274a of a second high-frequency electrode 274. A barrier portion 278a is formed outside the fluid discharge groove 276a at a position higher than the continuous electrode 274a. That is, the barrier portion 278a of the detachable side holding portion 224 is closer to the main body side holding section 222 than the surface of the continuous electrode 274a.

A vapor discharge groove 276b is annularly formed outside a discrete electrode 274b of the second high-frequency electrode 274. This fluid discharge groove 276b is in communication with the above-mentioned fluid discharge groove 276a. A barrier portion 278b is formed outside the fluid discharge groove 276b at a position higher than the surface of the discrete electrode 274b. That is, the barrier portion 278b of the detachable side holding portion 224 is closer to the main body side holding section 222 than the surface of the discrete electrode 274b.

It is to be noted that the surfaces of the barrier portions 278a, 278b are substantially flush with each other.

Furthermore, the fluid discharge grooves 276a, 276b are in communication with a fluid discharge passage 280 of the head portion 264 and an electro-conductive shaft 262. This fluid discharge passage 280 is in communication with a second fluid flow path (a fluid passage) 246b of an electro-conductive pipe 236. Moreover, a shaft 204 or a handle 202 is provided with a fluid discharge port (not shown) from which the fluid passed through the second fluid flow path 246b is discharged to the outside.

A function of a treatment system 10 according to this embodiment will now be explained.

The same function of the treatment system 10 according to this embodiment as the function in the ninth embodiment is omitting an explanation thereof.

As shown in FIG. 21B, in a state in which the main body side holding portion 222 is closed with respect to the detachable side holding portion 224, a holding section 206 and the shaft 204 of an electro-surgical device 12b are inserted into an abdominal cavity through, for example, an abdominal wall. The main body side holding portion 222 of the electro-surgical device 12b and the detachable side holding portion 224 are opposed to a living tissue to be treated.

The living tissue as a treatment target is held between the main body side holding portion 222 and the detachable side holding portion 224. At this time, the living tissue as a treatment target contacts the first high-frequency electrode 254 and the second high-frequency electrode 274. Tissues around the living tissue as a treatment target are closely contacted with the barrier portions 258a, 258b, 278a and 278b of the main body side holding portion 222 and the detachable side holding portion 224.

In this state, a foot switch and a hand switch are operated. An energy source 14 supplies energy to the first high-frequency electrode 254 and the second high-frequency electrode 274 via a cable 28, respectively.

The first high-frequency electrode 254 conducts a high-frequency current between the first high-frequency electrode 254 and the second high-frequency electrode 274 via the living tissue. Thus, the living tissue between the first high-frequency electrode 254 and the second high-frequency electrode 274 is heated.

Then, a fluid such as high-temperature vapor or liquid is generated from the heated part of the living tissue. Here, when the first high-frequency electrode 254 is fixed to the main body side holding portion 222, the surface of the first high-frequency electrode 254 exposed on the side of the detachable side holding portion 224 is positioned slightly lower than the barrier portions 258a, 258b of the main body side holding portion 222. In the same manner, when the second high-frequency electrode 274 is fixed to the detachable side holding portion 224, the surface of the second high-frequency electrode 274 exposed on the side of the main body side holding portion 222 is positioned slightly lower than the barrier portions 278a, 278b of the detachable side holding portion 224. Thus, the barrier portions 258a, 258b of the main body side holding portion 222 and the barrier portions 278a, 278b of the detachable side holding portion 224 serve as dams to bring the fluid generated from the living tissue by electric conduction between the first high-frequency electrode 254 and the second high-frequency electrode 274 into the fluid discharge grooves 256a, 256b, 276a and 276b and prevent the leakage of the fluid.

Then, in a state in which the main body side holding portion 222 is closed with respect to the detachable side holding portion 224, the barrier portions 258a, 258b of the main body side holding portion 222 are in contact with the barrier portions 278a, 278b of the detachable side holding portion 224, such that the fluid generated from the living tissue flows into the fluid discharge grooves 256a, 256b, 276a and 276b.

Subsequently, the fluid flown into the fluid discharge grooves 256a, 256b at the main body side holding portion 222 is passed toward the handle 202 inside a pusher 244 for the cutter through the first fluid flow path 246a in which the cutter 242 is disposed, and discharged to the outside of the electro-surgical device 12b.

On the other hand, the fluid flown into the fluid discharge grooves 276a, 276b at the detachable side holding portion 224 is passed toward the handle 202 through the fluid discharge passage 280 and the second fluid flow path 246b and discharged to the outside of the electro-surgical device 12b.

As explained above, according to the embodiment, the following effect can be obtained.

The same effects of the treatment system 10 according to this embodiment as the effects in the ninth embodiment are omitting an explanation thereof.

When a high-frequency current is applied by the electro-surgical device 12b to the living tissue as a treatment target held by the holding section 206, the barrier portions 258a, 258b of the main body side holding portion 222 and the barrier portions 278a, 278b of the detachable side holding portion 224 can be closely contacted with the living tissue. Thus, the fluid generated from the living tissue as a treatment target can be introduced into the first fluid flow path 246a through the vapor discharge groove 256a between the continuous electrode 254a of the first high-frequency electrode 254 and the barrier portion 258a and through the vapor discharge groove 256b between the discrete electrodes 254b and the barrier portion 258b, even if the fluid flows toward the barrier portions 258a, 258b of the main body side holding portion 222 and the barrier portions 278a, 278b of the detachable side holding portion 224. Further, the fluid can be introduced into the second fluid flow path 246b through the fluid discharge groove 276a between the continuous electrode 274a of the second high-frequency electrode 274 and the barrier portion 278a and through the fluid discharge groove 276b between the discrete electrode 274b and the barrier portion 278b.

In consequence, it can be prevented that the peripheral tissue other than the target tissue is influenced by the fluid generated from the portion to which the high-frequency current has been conducted during the treatment of the living tissue. That is, the position influenced during the treatment of the living tissue can be limited to the living tissue where the high-frequency current has been conducted between the first high-frequency electrode 254 and the second high-frequency electrode 274.

In addition, in the structure described in this embodiment, the barrier portions 258a, 258b are provided around the fluid discharge grooves 256a, 256b on the outer periphery of the first high-frequency electrode 254, and the barrier portions 278a, 278b are provided around the fluid discharge grooves 276a, 276b on the outer periphery of the second high-frequency electrode 274, in order to prevent thermal spread. Moreover, as described in the sixth embodiment (see FIGS. 16B to 16D), it is also preferable to provide a structure such as a cooling plate 104 or a thin flexible sheet-like member 114 made of a silicone material capable of indirectly cooling off the living tissue or a fluid such as vapor.

### [Thirteenth Embodiment]

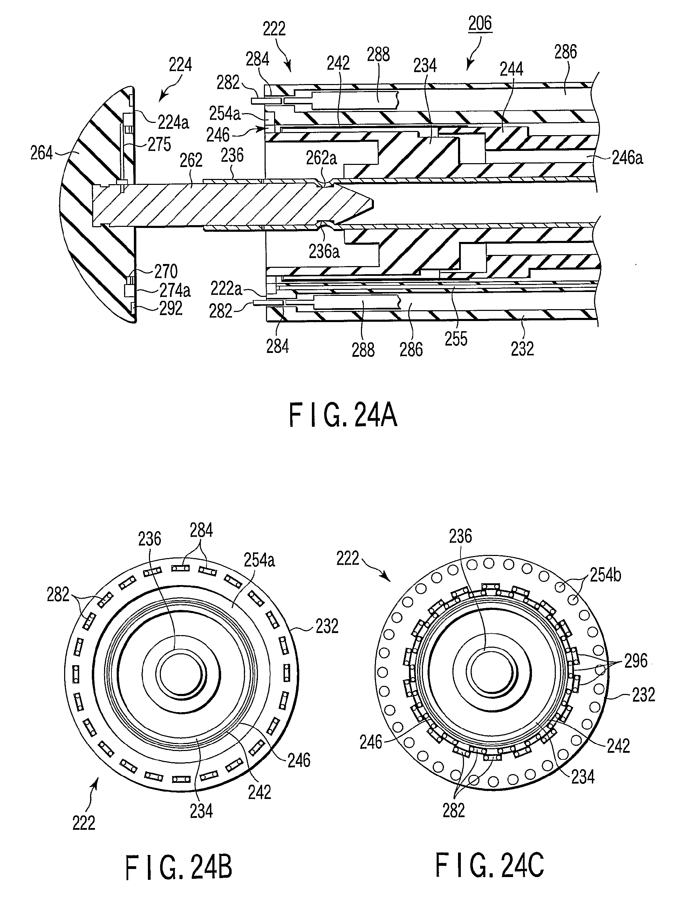

A thirteenth embodiment will now be explained with reference to FIGS. 24A to 24C. This embodiment is a modification of the seventh to ninth and twelfth embodiments, and like reference numbers denote members equal to those explained in the seventh to ninth and twelfth embodiments, thereby omitting a detailed explanation thereof.

As shown in FIGS. 24A and 24B, a plurality of discrete electrodes 254b (see FIG. 21D) described in the ninth embodiment are eliminated from a main body side holding portion 222, and a plurality of slots 284 for discharging surgical staples (maintaining member, second jointing member, applying portion) 282 are formed instead. The plurality of slots 284 are formed outside a continuous electrode 254a at intervals on a substantially annular track which is substantially concentric with, for example, the continuous electrode 254a.

As shown in FIG. 24A, a sled guide groove 286 is formed inside a cylindrical member 232 of the main body side holding portion 222. An actuation sled 288 is slidably provided in the sled guide groove 286. The actuation sled 288 is disposed parallel to a cutter 242 and a pusher 244 for the cutter, and the movement of this actuation sled 288 can be operated, in the same manner as a cutter driving lever 214, by a handle 202 using a staple operating unit (not shown) driven substantially parallel to the cutter 242 and the pusher 244 for the cutter. Therefore, the main body side holding portion 222 also functions as a cartridge of the staples 282.

Discrete electrodes 274b are eliminated from a detachable side holding portion 224, and deforming grooves 292 of the staples 282 are provided instead. That is, a plurality of staple deforming grooves 292 are formed in a holding surface 224a of the detachable side holding portion 224. Each of the staple deforming grooves 292 is formed with, for example, a substantially arc-shaped bottom so that a pair of legs of the staple 282 can be folded inwards and deformed. Therefore, the detachable side holding portion 224 also functions as an anvil of the staple 282.

It is to be noted that the slots 284 at the distal end of the main body side holding portion 222 are formed opposite to the staple deforming grooves 292 of the detachable side holding portion 224.

That is, this treatment system 10 is equipped with an electro-surgical device 12b for performing a physical treatment using the continuous electrodes 254a, 274a and also performing a mechanical treatment using the staples.

A function of a treatment system 10 according to this embodiment will now be explained.

As described in the ninth embodiment, a living tissue L_{T} as a treatment target is held between the main body side holding portion 222 and the detachable side holding portion 224. At this time, the living tissue L_{T} contacts the continuous electrodes 254a, 274a.

In this state, a foot switch or a hand switch is operated. Energy is respectively applied to the continuous electrodes 254a, 274a from an energy source 14. Then, the living tissue L_{T} between the continuous electrodes 254a, 274a is heated.

While the living tissue L_{T} as a treatment target is held between the main body side holding portion 222 and the detachable side holding portion 224, tissues targeted for anastomosis are interposed between the slots 284 of the main body side holding portion 222 and the staple deforming grooves 292 of the detachable side holding portion 224. In this state, the staple operating unit (not shown) of the handle 202 is operated. Then, the actuation sled 288 moves forward. Here, as shown in FIG. 24A, the actuation sled 288 moves forward along the sled guide groove 286.

When the actuation sled 288 moves forward, the staple 282 in the slot 134 projects and is inserted into the tissues targeted for anastomosis. The pair of legs of the inserted staple 282 penetrates the living tissues L_{T}. Then, the legs are folded inwards by the deforming groove 292 of the detachable side holding portion 224. Thus, the tissues targeted for anastomosis are anastomosed to each other by the staples 282. As a result, the living tissues L_{T} are discretely jointed to each other in a mechanical manner.

As explained above, according to the embodiment, the following effect can be obtained.

The continuous electrode 254a, 274a are disposed in the main body side holding portion 222 and the detachable side holding portion 224, respectively. The staple 282 is discharged from the slot 284 outside the continuous electrode 254a of the main body side holding portion 222, and the pair of legs of the staple 282 is folded inwards in the staple deforming grooves 292 outside the continuous electrode 274a of the detachable side holding portion 224. The living tissues L_{T} between the continuous electrode 254a of the main body side holding portion 222 and the continuous electrode 274a of the detachable side holding portion 224 can be heated, denatured, and continuously jointed to each other. Consequently, the living tissues L_{T} can be closely contacted with each other or sealed with each other. Moreover, the living tissues L_{T} can be discretely jointed mechanically by the staples 282 in the vicinity of the outer side of the part where these living tissues are continuously jointed.

Therefore, the parts of the living tissues L_{T} jointed by the staples 282 serve to draw the living tissues L_{T} close to each other and keep them closely contacted with each other. Thus, an inter-network is produced between the living tissues L_{T} such that the closely contacted living tissues L_{T} more easily exercise their tissue regeneration power and can be regenerated earlier.

Although the staples 282 are used to mechanically joint the living tissues L_{T} to each other in the example explained in this embodiment, it is also preferable that, for example, a living tissue adhesive is used to joint the living tissues L_{T} to each other.

Moreover, as shown in FIG. 24C, it is also preferable to provide a structure in which slots 296 for discharging the staples are substantially annularly and densely formed instead of the continuous electrode 254a of the main body side holding portion 222.

Additional advantages and modifications will readily occur to those skilled in the art. Therefore, the invention in its broader aspects is not limited to the specific details and representative embodiments shown and described herein. Accordingly, various modifications may be made without departing from the scope of the general inventive concept as defined by the appended claims.

## Claims

1. A treatment device to treat at least two living tissues (L_{T}), comprising:
a pair of holding members (52, 54) respectively having holding surfaces (62a, 66a) to hold the at least two living tissues (L_{T});
a cutter guide groove (94, 96; 246) formed in each of the holding surfaces (62a, 66a), configured to guide a cutter (84; 242) therealong in order to cut the living tissues (L_{T});
a sealing member (56a; 132; 254a, 274a) disposed on the holding surface (62a, 66a) of at least one of the holding members (52, 54), configured to join and seal contact areas of the at least two living tissues (L_{T}) when an energy is applied thereto while the desired contact areas of the at least two living tissues (L_{T}) are being contacted to each other; and
a maintaining member (56b-56o; 132; 254b-254f) disposed on the holding surface and configured to maintain living tissues (L_{T}) located on a periphery of an area jointed and sealed by the sealing member (56a; 132; 254a, 274a), in the contact state when the energy is applied to the living tissues (L_{T}),
wherein the maintaining member (56b-56o; 132; 254b-254f) includes a plurality of parts arranged to be separate from each other so as to exert a physical action to the living tissues (L_{T}),
the sealing member (56a; 254a, 274a) is a seamlessly and continuously formed sealing member disposed on the holding surface so as to surround the cutter guide groove (94, 96; 246) and is configured to join and seal seamlessly the contact areas, and
the parts of the maintaining member (56b-56o; 132; 254b-254f) are discretely disposed on an outer edge side of at least one of the holding members (52, 54) with respect to the sealing member (56a; 132; 254a, 274a),
**characterized in that**
the sealing member (56a; 132; 254a, 274a) includes a portion configured to seal the living tissues with the energy from at least one of a high frequency electrode and a heater and **in that** the maintaining member (56b-56o; 132; 254b-254f) includes a portion configured to maintain the contact between the living tissues (L_{T}) with the energy from at least one of a high frequency electrode and a heater.

2. The treatment device according to claim 1, **characterized in that**
the sealing member (56a; 132; 254a, 274a) includes an electrode (56a) disposed in at least one of the holding surfaces (62a, 66a), and
the maintaining member (56b-56o; 132; 254b-254f) includes a plurality of electrodes (56b) disposed in at least one of the holding surfaces (62a, 66a).

3. The treatment device according to claim 1, **characterized in that**
the sealing member (56a; 132; 254a, 274a) includes an electrode (56a) disposed in at least one of the holding surfaces (62a, 66a), and
the maintaining member (56b-56o; 132; 254b-254f) includes a plurality of electrodes (56b) branching from the electrode (56a) of the sealing member (56a; 254a, 274a).

4. The treatment device according to any one of claims 1 to 3, **characterized by** further comprising:
at least one barrier portion (98) which is provided adjacently to the sealing member (56a; 254a, 274a) and the maintaining member (56b-56o; 132; 254b-254f), the barrier portion (98) being configured to project from the holding surface (62a, 66a).

5. The treatment device according to any one of claims 1 to 3, **characterized in that** the holding members (52, 54) include a flow path (246a, 246b), a groove or a conduit which passes a fluid generated from the living tissues (L_{T}) held by the holding members (52, 54).

6. The treatment device according to claim 1, **characterized in that** the sealing member (56a; 132; 254a, 274a) and the maintaining member (56b-56o; 132; 254b-254f) are formed into one continuous wave form in at least one of the holding surfaces (62a, 66a).

## Patentansprüche

1. Behandlungsvorrichtung zum Behandeln von mindestens zwei lebenden Geweben (L_{T}), aufweisend:
ein Paar Halteelemente (52, 54) mit jeweiligen Halteflächen (62a, 66a) zum Halten der mindestens zwei lebenden Gewebe (L_{T});
eine Schnittführungsnut (94, 96; 246), die in jeder der Halteflächen (62a, 66a) gebildet und dazu eingerichtet ist, ein Messer (84; 242) zum Schneiden der lebenden Gewebe (L_{T}) entlang dieser zu führen;
ein Versiegelungselement (56; 132; 254a, 274a), das auf der Haltefläche (62a, 66a) von mindestens einem der Halteelemente (52, 54) angeordnet und dazu eingerichtet ist, Kontaktbereiche der mindestens zwei lebenden Gewebe (L_{T}) zu verbinden und zu versiegeln wenn Energie daran angelegt wird während die gewünschten Kontaktbereiche der mindestens zwei lebenden Gewebe (L_{T}) miteinander in Verbindung gebracht werden; und
ein Beibehaltungselement (56b-56o; 132; 254b-254f), das auf der Haltefläche angeordnet und dazu eingerichtet ist, lebende Gewebe (L_{T}), die an einem Umfang eines Bereichs, der durch das Versiegelungselement (56a; 132; 254a, 274a) verbunden und versiegelt wurde, angeordnet ist, in dem Kontaktzustand beizubehalten, wenn die Energie an die lebenden Gewebe (L_{T}) angelegt wird,
wobei das Beibehaltungselement (56b-56o; 132; 254b-254f) eine Mehrzahl von Teilen enthält, die angeordnet sind um voneinander beabstandet zu sein, um eine physikalische Aktion auf die lebenden Gewebe (L_{T}) auszuüben,
das Versiegelungselement (56a; 254a, 274a) ein übergangslos und kontinuierlich gebildetes Versiegelungselement ist, das auf der Haltefläche angeordnet ist, um die Schnittführungsnut (94, 96; 246) zu umgeben, und dazu eingerichtet ist, die Kontaktbereiche übergangslos zu verbinden und zu versiegeln, und
die Teile des Beibehaltungselements (56b-56o; 132; 254b-254f) getrennt an einer äußeren Kantenseite von mindestens einem der Halteelemente (52, 54) hinsichtlich des Versiegelungselements (56a; 132; 254a, 274a) angeordnet sind,
**dadurch gekennzeichnet, dass**
das Versiegelungselement (56a; 132; 254a, 274a) einen Teil enthält, der dazu eingerichtet ist, die lebenden Gewebe mit der Energie von einer Hochfrequenzelektrode und/oder einem Erhitzer zu versiegeln, und dass das Beibehaltungselement (56b-56o; 132; 254b-254f) einen Teil enthält, der dazu eingerichtet ist, mit der Energie von der Hochfrequenzelektrode und/oder dem Erhitzer den Kontakt zwischen den lebenden Geweben (L_{T}) beizubehalten.

2. Behandlungsvorrichtung nach Anspruch 1, **dadurch gekennzeichnet, dass**
das Versiegelungselement (56a; 132; 254a, 274a) eine Elektrode (56a) enthält, die in mindestens einer der Halteflächen (62a, 66a) angeordnet ist, und
das Beibehaltungselement (56b-56o; 132; 254b-254f) eine Mehrzahl von Elektroden (56b) enthält, die in mindestens einer der Halteflächen (62a, 66a) angeordnet ist.

3. Behandlungsvorrichtung nach Anspruch 1, **dadurch gekennzeichnet, dass**
das Versiegelungselement (56a; 132; 254a, 274a) eine Elektrode (56a) enthält, die in mindestens einer der Halteflächen (62a, 66a) angeordnet ist, und
das Beibehaltungselement (56b-56o; 132; 254b-254f) eine Mehrzahl von Elektroden (56b) enthält, die von der Elektrode (56a) des Versiegelungselements (56a; 254a, 274a) abzweigen.

4. Behandlungsvorrichtung nach einem der Ansprüche 1 bis 3, **dadurch gekennzeichnet, dass** es ferner aufweist:
mindestens ein Grenzteil (98), das neben dem Versiegelungselement (56a; 254a, 274a) und dem Beibehaltungselement (56b-56o; 132; 254b-254f) bereitgestellt ist, wobei das Grenzteil (98) dazu eingerichtet ist, von der Haltefläche (62a, 66a) hervorzustehen.

5. Behandlungsvorrichtung nach einem der Ansprüche 1 bis 3, **dadurch gekennzeichnet, dass** die Halteelemente (52, 54) einen Flusspfad (246a, 246b), eine Nut oder eine Leitung enthalten, der/die von dem lebenden Gewebe (L_{T}), das von den Halteelementen (52, 54) gehaltenen wird, erzeugte Flüssigkeit abführt.

6. Behandlungsvorrichtung nach Anspruch 1, **dadurch gekennzeichnet, dass** das Versiegelungselement (56a; 132; 254a, 274a) und das Beibehaltungselement (56b-56o; 132; 254b-254f) in einer kontinuierlichen Wellenform in mindestens einer der Halteflächen (62a, 66a) gebildet sind.

## Revendications

1. Dispositif de traitement pour traiter au moins deux tissus vivants (L_{T}), comprenant :
une paire d'éléments de prise (52, 54) comportant respectivement des surfaces de prise (62a, 66a) pour tenir les au moins deux tissus vivants (L_{T}) ;
une rainure (94, 96 ; 246) de guidage d'élément tranchant formée dans chacune des surfaces de prise (62a, 66a), configurée pour guider un élément tranchant (84 ; 242) le long de celle-ci afin de couper les tissus vivants (L_{T}) ;
un élément d'étanchéité (56a ; 132 ; 254a, 274a) disposé sur les surfaces de prise (62a, 66a) d'au moins l'un des éléments de prise (52, 54), configuré pour relier et sceller des zones de contact des au moins deux tissus vivants (L_{T}) lorsqu'une énergie est appliquée sur ceux-ci alors que les zones de contact souhaitées des au moins deux tissus vivants (L_{T}) sont en contact l'une avec l'autre ; et
un élément de maintien (56b à 56o ; 132 ; 254b à 254f) disposé sur la surface de prise et configuré pour maintenir les tissus vivants (L_{T}) placés sur une périphérie d'une zone reliée et scellée par l'élément d'étanchéité (56a ; 132 ; 254a, 274a), dans l'état de contact lorsque l'énergie est appliquée aux tissus vivants (L_{T}),
dans lequel l'élément de maintien (56b à 56o ; 132 ; 254b à 254f) comprend une pluralité de parties agencées pour être séparées les unes des autres de façon à exercer une action physique sur les tissus vivants (L_{T}),
l'élément d'étanchéité (56a ; 132 ; 254a, 274a) est un élément d'étanchéité formé de manière homogène et continue disposé sur la surface de prise de façon à entourer la rainure (94, 96 ; 246) de guidage d'élément tranchant et est configuré pour relier et sceller de manière homogène les zones de contact, et
les parties de l'élément de maintien (56b à 56o ; 132 ; 254b à 254f) sont disposées séparément sur un côté de bord externe d'au moins l'un des éléments de prise (52, 54) par rapport à l'élément d'étanchéité (56a ; 132 ; 254a, 274a),
**caractérisé en ce que**
l'élément d'étanchéité (56a ; 132 ; 254a, 274a) comprend une partie configurée pour sceller les tissus vivants avec l'énergie provenant d'au moins l'un parmi une électrode haute fréquence et un dispositif chauffant et **en ce que** l'élément de maintien (56b à 56o ; 132 ; 254b à 254f) comprend une partie configurée pour maintenir le contact entre les tissus vivants (L_{T}) avec l'énergie provenant d'au moins l'un parmi une électrode haute fréquence et un dispositif chauffant.

2. Dispositif de traitement selon la revendication 1, **caractérisé en ce que**
l'élément d'étanchéité (56a ; 132 ; 254a, 274a) comprend une électrode (56a) disposée dans au moins l'une des surfaces de prise (62a, 66a), et
l'élément de maintien (56b à 56o ; 132 ; 254b à 254f) comprend une pluralité d'électrodes (56b) disposées dans au moins l'une des surfaces de prise (62a, 66a).

3. Dispositif de traitement selon la revendication 1, **caractérisé en ce que**
l'élément d'étanchéité (56a ; 132 ; 254a, 274a) comprend une électrode (56a) disposée dans au moins l'une des surfaces de prise (62a, 66a), et
l'élément de maintien (56b à 56o ; 132 ; 254b à 254f) comprend une pluralité d'électrodes (56b) s'embranchant à partir de l'électrode (56a) de l'élément d'étanchéité (56a ; 132 ; 254a, 274a).

4. Dispositif de traitement selon l'une quelconque des revendications 1 à 3, **caractérisé en ce qu'**il comprend en outre :
au moins une partie barrière (98) qui est prévue de manière adjacente à l'élément d'étanchéité (56a ; 132 ; 254a, 274a) et l'élément de maintien (56b à 56o ; 132 ; 254b à 254f), la partie barrière (98) étant configurée pour faire saillie depuis la surface de prise (62a, 66a).

5. Dispositif de traitement selon l'une quelconque des revendications 1 à 3, **caractérisé en ce que** les éléments de prise (52, 54) comprennent un trajet d'écoulement (246a, 246b), une rainure ou un conduit qui laisse passer un fluide généré par les tissus vivants (L_{T}) tenus par les éléments de prise (52, 54).

6. Dispositif de traitement selon la revendication 1, **caractérisé en ce que** l'élément d'étanchéité (56a ; 132 ; 254a, 274a) et l'élément de maintien (56b à 56o ; 132 ; 254b à 254f) sont formés selon une forme d'onde continue dans au moins l'une des surfaces de prise (62a, 66a).
